(19) Europäisches Patentamt
European Patent Office
Office européen des brevets

(11) EP 4 011 444 A1

(12) EUROPEAN PATENT APPLICATION
published in accordance with Art. 153(4) EPC

(43) Date of publication:
**15.06.2022 Bulletin 2022/24**

(21) Application number: **20848934.4**

(22) Date of filing: **30.07.2020**

(51) International Patent Classification (IPC):
***A61P 1/04*** (2006.01) ***A61P 1/16*** (2006.01)
***A61P 3/04*** (2006.01) ***A61P 35/00*** (2006.01)
***C08F 8/14*** (2006.01) ***C08F 290/06*** (2006.01)
***C08G 65/334*** (2006.01) ***C08F 293/00*** (2006.01)
***A61K 47/60*** (2017.01) ***A61K 31/19*** (2006.01)

(52) Cooperative Patent Classification (CPC):
**A61K 31/19; A61K 47/60; A61P 1/04; A61P 1/16; A61P 3/04; A61P 35/00; C08F 8/14; C08F 290/06; C08F 293/00; C08G 65/334**

(86) International application number:
**PCT/JP2020/029239**

(87) International publication number:
**WO 2021/024906 (11.02.2021 Gazette 2021/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.08.2019 JP 2019143619**

(71) Applicant: **University of Tsukuba**
**Ibaraki 305-8577 (JP)**

(72) Inventors:
- **NAGASAKI, Yukio**
  **Tsukuba-shi, Ibaraki 305-8577 (JP)**
- **SHASHNI, Babita**
  **Tsukuba-shi, Ibaraki 305-8577 (JP)**
- **VONG, Binh Long**
  **Tsukuba-shi, Ibaraki 305-8577 (JP)**
- **OKADA, Ryusaku**
  **Tsukuba-shi, Ibaraki 305-8577 (JP)**
- **TAJIKA, Yuya**
  **Tsukuba-shi, Ibaraki 305-8577 (JP)**
- **LEE, Yaroslav**
  **Tsukuba-shi, Ibaraki 305-8577 (JP)**

(74) Representative: **Beckmann, Claus**
**Kraus & Weisert**
**Patentanwälte PartGmbB**
**Thomas-Wimmer-Ring 15**
**80539 München (DE)**

# (54) HYDROPHILIC-HYDROPHOBIC COPOLYMER CARRYING SHORT-CHAIN FATTY ACID ESTER

(57) [Object]

To provide a derivative of a short chain fatty acid that can exhibit physiological functions inherent in the short chain fatty acid.

[Solution]

Provided is a block or graft copolymer including a hydrophobic segment of a repeat unit containing a short chain fatty acid ester that is hydrolyzable by esterase in vivo and a hydrophilic segment including a poly(ethylene glycol) chain. These copolymers are effective in the treatment or therapeutic treatment of various diseases or disorders, including cancer.



Processed by Luminess, 75001 PARIS (FR)

## Description

### Technical Field

The present invention relates to a block copolymer or a graft copolymer containing a hydrophobic portion carrying a short chain fatty acid ester and a poly(ethylene glycol) chain as a hydrophilic portion, and use thereof as a nanomedicine material.

### Background Art

Short chain fatty acids, including acetic acid, propionic acid and butyric acid (or butanoic acid), have been reported to have a variety of physiological functions, such as immunosuppressive ability, suppression of liver fibrosis, obesity suppressive ability and anticancer ability depending on their chain length (e.g., Non-Patent Literature 1). Short chain fatty acids having the physiological functions as described above are produced from saccharides by intestinal flora, but are not necessarily sufficient and are expected to be supplied sufficiently.

However, short chain fatty acids are not only limited in terms of administration method due to their solubility and odor, but also are fast-metabolized due to their low molecular weight, and, additionally, are difficult to treat because their physiological functions are changed by association. Therefore, some of short chain fatty acids are provided as supplements or the like, but it is no exaggeration to say that there is no effective administration method or formulation capable of exerting their inherent physiological functions.

### Citation List

### Non Patent Literature

Non-Patent Literature 1: Takashi Sakata, Hirohumi Ichikawa, Physiological Actions of Short-Chain Fatty Acids, Journal of Japan Oil Chemists' Society, Vol. 46, 1205-1212 (1997)

### Summary of Invention

### Technical Problem

An object of the present invention is to provide a means capable of effectively administering and delivering a short chain fatty acid into a living body.

### Solution to Problem

The present inventors have designed and proposed so far polymerized drug systems in which a drug to be delivered is carried on a polymeric compound, which is associative and can self-assemble in an aqueous medium, to modify drug delivery characteristics (e.g., WO 2009/133647 and WO 2016/052463). The present inventors have repeated studies, on the assumption that successful use of any polymerized drug system to achieve the object described above will contribute to the solution of the above-described problems.

As a result, it has been confirmed that a block copolymer containing or carrying a hydrophobic portion that carrys a short chain fatty acid ester and a poly(ethylene glycol) chain, or a graft copolymer containing or carrying hydrophobic portions that carry a short chain fatty acid ester and poly(ethylene glycol) chains, or a nanoparticle or a nano-sized polymeric micelle formed upon self-assembling through association thereof in water can cure or correct disadvantages or defects in the delivery of the short chain fatty acid described above.

Accordingly, main aspects provided in accordance with the present invention can include the following aspects.

Aspect 1: A hydrophilic-hydrophobic copolymer including:

(1) a hydrophobic segment derived from a repeat unit, represented by Formula I:

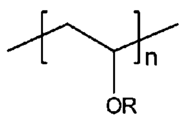

(I)

Where in Formula I, R is $-(C=O)R^1$ or a hydrogen atom, in which $R^1$ is an unsubstituted or substituted linear or branched alkyl having from 1 to 7 carbon atoms (when $R^1$ is substituted, a substituent is unsubstituted or substituted phenyl, and a substituent of the substituted phenyl is one or more halogen, hydroxyl, or methyloxy), and the hydrogen atom, if present, exists in such a number that is 30% or less, preferably 20% or less, more preferably 10% or less, most preferably 0% of n, where n is an integer from 5 to 1000, preferably from 10 to 1000, more preferably from 15 to 1000, or from 30 to 1000; and

(2) a hydrophilic segment including a poly(ethylene glycol) chain that is:

(i) represented by Formula IIa:

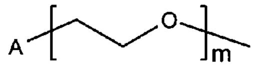

(IIa)

where in Formula IIa, A is unsubstituted or substituted $C_1$-$C_{12}$ alkyloxy, and, when A is substituted, a substituent is a formyl group, formula R'R"CH- group, a phenylamino group or phenethyl amino group, a phenyl group, or a methoxyphenyl group, where R' and R" are independently $C_1$-$C_4$ alkyloxy, or R'and R" are taken together to form $-OCH_2CH_2O-$, $-O(CH_2)_3O-$ or $-O(CH_2)_4O-$, and m is an integer from 2 to 500, preferably from 10 to 300, more preferably from 20 to 200, or

(ii) derived from a repeat unit represented by Formula IIb:

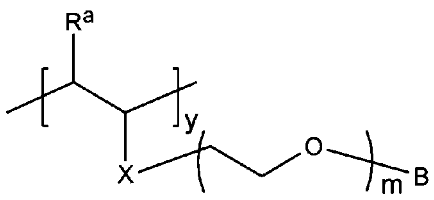

(IIb)

where in Formula IIb,

$R^a$ is a hydrogen atom or a carboxy group,
X is C (=O)O or C(=O)NH when $R^a$ is a carboxy group, or is O or NH when $R^a$ is a hydrogen atom,
B is $A-CH_2CH_2$, and A and m are each as defined above, and
y is an integer from 1 to 300, preferably from 2 to 150, more preferably from 5 to 100,
wherein the (1) hydrophobic segment and the (2) hydrophilic segment of (i) are each presented as blocks, and
wherein each member of the repeat units including the (1) hydrophobic segment and the (2) hydrophilic segment of (ii) exists randomly with each other.

Aspect 2: The hydrophilic-hydrophobic copolymer according to Aspect 1, which is associated and self-assembled in water to form nanoparticles or nano-sized polymeric micelles.

Aspect 3: The hydrophilic-hydrophobic copolymer according to Aspect 1 or 2,

wherein the copolymer including the (1) hydrophobic segment derived from a repeat unit represented by Formula (I) and the (2) hydrophilic segment of (i) Formula IIa is a block copolymer represented by Formula BC:

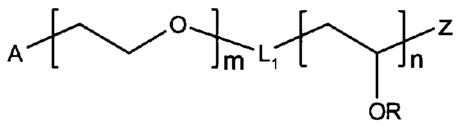

(BC)

where in Formula BC, A, R, m, and n are each as defined above, $L_1$ represents a direct bond or a divalent linking group, Z is a hydrogen atom, SH, S (C=S)-Ph, $S(=S)OCH_2CH_3$, a hydroxyl group, a $C_1$-$C_6$ alkyloxy group or an aryl-$C_1$-$C_2$ aryloxy group, and

wherein the copolymer including the (1) hydrophobic segment derived from a repeat unit represented by Formula (I) and the (2) hydrophilic segment of (ii) Formula IIb is a graft copolymer represented by Formula GC:

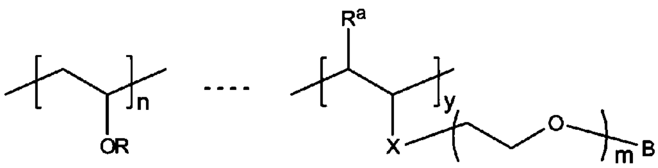

(GC)

Where in Formula GC, R, $R^a$, B, X, m, n, and y are each as defined above.

Aspect 4: A nanoparticle formed, in an aqueous medium, from the hydrophilic-hydrophobic copolymer according to any one of Aspects 1 to 3.

Aspect 5: A pharmaceutical formulation comprising, as an active ingredient, the hydrophilic-hydrophobic copolymer according to any one of Aspects 1 to 3 or the nanoparticle described in claim 4.

Aspect 6: The pharmaceutical formulation according to Aspect 5 for use in preventing or treating cancer, suppressing obesity, preventing or treating ulcerative colitis or non-alcoholic fatty liver (or suppressing liver fibrosis), preventing or treating diabetes, enhancing radiation in radiation therapy, or preventing or treating hyperammonemia.

Aspect 7: The hydrophilic-hydrophobic copolymer according to any one of claims 1 to 3 for use in preventing or treating cancer, suppressing obesity, preventing or treating ulcerative colitis or non-alcoholic fatty liver, preventing or treating diabetes, enhancing radiation in radiation therapy, or preventing or treating hyperammonemia.

Aspect 8: The nanoparticle according to Aspect 4 for use in preventing or treating cancer, suppressing obesity, preventing or treating ulcerative colitis or non-alcoholic fatty liver (or suppressing liver fibrosis), preventing or treating diabetes, enhancing radiation in radiation therapy, or preventing or treating hyperammonemia.

Aspect 9: A method for preventing or treating cancer, suppressing obesity, preventing or treating ulcerative colitis or non-alcoholic fatty liver (or suppressing liver fibrosis), preventing or treating diabetes, enhancing radiation in radiation therapy, or preventing or treating hyperammonemia, the method including administering the hydrophilic-hydrophobic copolymer according to any one of Aspects 1 to 3 to a patient in need thereof.

Aspect 10: A method for preventing or treating cancer, suppressing obesity, preventing or treating ulcerative colitis or non-alcoholic fatty liver (or suppressing liver fibrosis), preventing or treating diabetes, enhancing radiation in radiation therapy, or preventing or treating hyperammonemia, the method including administering the hydrophilic-hydrophobic copolymer according to Aspect 4 to a patient in need thereof.

Advantageous Effects of Invention

The hydrophilic-hydrophobic copolymer or the nanoparticle or polymeric micelle thereof according to the present invention can be delivered to a local site in a living body, when administered to a mammal, including a human, to enzymatically hydrolyze short chain fatty acid ester bonds in the hydrophobic segment at the local site to slowly release the corresponding short chain fatty acid, and can eliminate or mitigate problems associated with the administration of the short chain fatty acid itself. As a result, there can be provided a copolymer capable of efficiently exerting various physiological functions inherent in short chain fatty acids locally or systemically in a living body of a mammal, and a nanoparticle thereof and a pharmaceutical formulation thereof.

Brief Description of Drawings

FIG. 1 is a $^1H$ NMR spectrum of N684 obtained in Production Example 3.
FIG. 2 is a $^1H$ NMR spectrum of N731 obtained in Production Example 4.
FIG. 3 is a $^1H$ NMR spectrum of N721 obtained in Production Example 5.
FIG. 4 is a $^1H$ NMR spectrum of N741 obtained in Production Example 6.
FIG. 5 is each of dynamic light scattering spectra of N684, N731, and N721 obtained in Production Example 7.
FIG. 6 is a dynamic light scattering spectrum of N741 obtained in Production Example 8.
FIG. 7 is graphical representation of cytotoxicity of vinyl ester nanoparticles (N684, N731, and N721) of Test Example 1.
FIG. 8 is a graphical representation of cytotoxicity of vinyl ester nanoparticles (N741) of Test Example 2.
FIG. 9 is a graphical representation of a body weight change of each test group mouse of Test Example 3. The data are on samples diluted 5 times, 10 times, 20 times, and 40 times from the left.
FIG. 10 is a graphical representation of results of confirmation (observation by the naked eye) of a number of lung cancer metastases of Test Example 3. The data are on water (control) and samples are diluted 5 times, 10 times, 20 times, 40 times, 80 times, and 160 times.
FIG. 11 is a graphical representation of results of measuring, under a microscope, a number of micrometastases that cannot be observed by the naked eye in the confirmation of the number of lung cancer metastases according to Test Example 3.
FIG. 12 is photographs (left) replacing H & E staining views of lung tissue views in Test Example 3 and a graphical representation (right) of a number of metastatic cancers determined from the views.
FIG. 13 is photographs (left) replacing H & E staining views of lung tissue views in Test Example 3 and a graphical representation (right) of an area of metastatic cancer determined from the views.
FIG. 14 is graphical representations of ALT, AST, LDH, and ALB levels in the blood according to Test Example 3.
FIG. 15 is photographs (left) replacing H & E staining views of the duodenum in Test Example 3 and a graphical representation (right) of a villus length of each test group.
FIG. 16 is photographs (left) replacing H & E staining views of the jejunum in Test Example 3 and a graphical representation (right) of a villus length of each test group.
FIG. 17 is photographs (left) replacing H & E staining views of the ileum in Test Example 3 and a graphical representation (right) of a villus length of each test group.
FIG. 18 is photographs (left) replacing H & E staining views of colorectal tissues in Test Example 3 and a graphical representation (right) of a villus length of each test group.
FIG. 19 is a graphical representation of a body weight change in Test Example 4.
FIG. 20 is a graphical representation of a disease activity index (DAI) of Test Example 5.
FIG. 21 is a graphical representation of a white blood cell count of each experimental animal of each treatment group (including a control) of Test Example 5.
FIG. 22 is graphical representation of liver and spleen weights of each experimental animal of each treatment group (including a control) of Test Example 6.
FIG. 23 is graphical representations of sample consumptions and body weight changes of experimental animals during testing of Test Example 7.
FIG. 24 is graphical representation of sample consumptions and body weight changes of experimental animals in Test (1) of Test Example 8.
FIG. 25 is a graphical representation of the results of a glucose resistance test in Test (2) of Test Example 8.
FIG. 26 is graphical representation of organ weights at the end of Test (3) of Test Example 8.
FIG. 27 is photographs replacing views illustrating histological analysis results of H & E stained intestinal tract in Test (4) of Test Example 8.
FIG. 28 is photographs replacing views illustrating histological analysis results of H & E stained pancreas tissues in Test (5) of Test Example 8.
FIG. 29 is a graphical representation illustrating the effect of radiation irradiation on a body weight change/volume change in Test (1) of Test Example 9.
FIG. 30 is a graphical representation illustrating the effect of radiation irradiation on a cancer volume change in Test (2) of Test Example 9.
FIG. 31 is a graphical representation of data on a cancer proliferation profile in Test (1) of Test Example 10.
FIG. 32 is a graphical representation of a cancer weight at the end of Test (2) of Test Example 10.
FIG. 33 is a graphical representation illustrating body weight changes of test animals in Test (3) of Test Example 10.
FIG. 34 is photographs replacing views illustrating a spheroid cancer cell growth suppressive effect related to a radiation enhancement effect in Test Example 11.
FIG. 35 is graphical representations of liver and spleen weights in Test (1) of Test Example 12.
FIG. 36 is a graphical representation of histological analysis results of HE stained liver in Test (2) of Test Example 12.
FIG. 37 is a graphical representation of histological analysis results of MT stained liver of Test (3) of Test Example 12.

FIG. 38 is a $^1H$ NMR spectrum of N821 obtained in Production Example 9.

FIG. 39 is a representation of size distribution of nanoparticles (Ph-BNP, N832) obtained in Production Example 10.

FIG. 40 is graphical representations of biochemical assay results of blood in Test (1) of Test Example 13.

FIG. 41 is photographs replacing histological analysis views of H & E stained liver of blood in Test (2) of Test Example 13.

FIG. 42 is graphical representations of results of Pharmacokinetic study (1) of Test Example 14.

FIG. 43 is a graphical representation of results of Pharmacokinetic study (2) of Test Example 14.

Detailed Description of Invention

The terms and the like described in relation to the present invention are used as having the meanings or contents that have been commonly used in the art unless otherwise mentioned. In general, the present invention may be additionally described below.

Short chain fatty acids can be produced from saccharides by intestinal flora of mammals, as described above, typically including acetic acid, propionic acid, and butyric acid (or butanoic acid), and, in some cases, including branched chain fatty acids such as isobutyric acid and isovaleric acid, which may be produced by the degradation of proteins containing certain branched chain amino acids, and even straight or branched chain fatty acids having up to 7 carbon atoms, which may exert similar functions to those of these fatty acids. Thus, when R in the hydrophobic segment represented by Formula I to provide a hydrophobic domain in the copolymer disclosed herein is $-(C{=}O)R^1$, examples of $R^1$ can include, but are not limited to, methyl, ethyl, propyl, isopropyl, butyl, isobutyl, heptyl, pentyl, and 3-methylbutyl. These groups may be substituted; when they are substituted, a substituent is unsubstituted or substituted phenyl preferably capable of binding to a carbon atom at a non-binding end; and a substituent of the substituted phenyl can be one or more halogen, hydroxy, or methyloxy. Also, alkyl moieties are described as $C_x$-$C_{xx}$ alkyloxy and the like mean straight or branched alkyl having from x to xx carbon atoms. R can be a hydrogen atom, and, here, R is 30% or less, preferably 20% or less, more preferably 10% or less, most preferably 0% (absent) of the total number of repeat units of n. The degree of preferability is a degree to more reliably form the hydrophobic domain or region when the copolymer is associated and self-assembled in water.

In the copolymer disclosed herein, the segment represented by Formula IIa to provide a hydrophilic segment can be a hydrophilic block, and, on the other hand, the hydrophobic segment represented by Formula I provides a hydrophobic block, and those segments can be a member of a hydrophilic-hydrophobic block copolymer, and,
each repeat unit represented by Formula IIb, which provides a hydrophilic segment, can be a member of a hydrophilic-hydrophobic random copolymer, which can be randomly present mutually with each hydrophobic segment represented in Formula I. "Mutually randomly" means that, if appropriate, they may be present in an alternating manner, for example, when n of Formula I and y of Formula IIb are close numerical values; for example, when a ratio of n of Formula I to y of Formula IIb is 30 or greater: 1, the hydrophobic segment may be substantially in a form such that a plurality of segments form a block.

The block copolymer and random copolymer can include other members, as long as a plurality of copolymers can be associated and self-assembled in water to form a so-called core-shell nanoparticle or polymeric micelle in which a hydrophobic segment is included in a core, and a hydrophilic segment is included in a shell. Typical examples of the copolymers disclosed herein can include, but are not limited to, copolymers represented by Formula BC or Formula GC as described above. For example, when $L_1$ in Formula BC represents a divalent linking group, the divalent linking group generally means a group containing up to 34, preferably 18, more preferably up to 10 carbon atoms, and optionally oxygen and nitrogen atoms. Specific examples of such linking groups can include the following groups:
a group selected from the groups represented by:

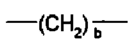 ,

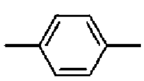 , 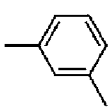 , 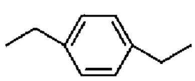 , 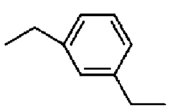 ,

or selected from the group consisting of: $-(CH_2)_cS-$, $-CO(CH_2)_cS-$, $-(CH_2)_cNH-$, $-(CH_2)_cCO-$, $-CO-$, $-OCOO-$, and $-CONH-$, where, each independently, b can be an integer from 2 to 6, and c is an integer from 1 to 5.

"Nano or nano-sized" in "nanoparticles or nano-sized polymeric micelles", in relation to the present invention, means that, when subjected to dynamic light scattering measurement (DLS) in water, nanoparticles or polymeric micelles have an average diameter in nanometer order, and that the average diameter is generally in the size from about 10 nm

to about 2000 nm, preferably from about 10 nm to about 500 nm, more preferably from about 25 nm to about 200 nm.

The above hydrophilic-hydrophobic copolymer can be produced in accordance with a per se known production method, with reference to the chemical structures defined. Conveniently, the hydrophilic-hydrophobic copolymer can be produced in accordance with either of the following methods.

The copolymer represented by typical Formula BC, as defined by (1) and (2)(i) above, is conveniently produced as follows, with reference to WO 2009/133647 and WO 2016/052463 indicated above.

First, a poly(ethylene glycol) (PEG) segment, can be produced by using a method involving providing a PEG derivative represented by:

Formula A-1:

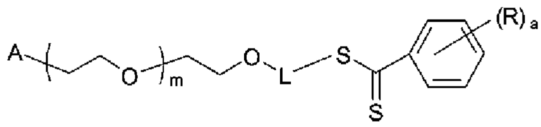

( A-1 )

or

Formula A-2:

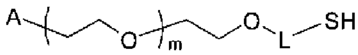

( A-2 )

(where A, L, and m are as defined above), then providing a short chain fatty acid ester of vinyl alcohol corresponding to the repeat unit of Formula I, and adding the latter to a unit of the former by a living radical polymerization reaction via reversible degenerative chain transfer. At this time, when L-S(C=S)Ph in A-1 is L-S(C=S) $OCH_2CH_3$ which will be described below, a copolymer of interest can be obtained more efficiently. Note that, when A has a phenylamino group or a phenethylamino group as a substituent, the substituent can be introduced by a reductive amination reaction of a formyl group of a compound corresponding to Formula IIa having the formyl group as a substituent with a corresponding amine.

The graft copolymer represented by typical Formula GC can be produced by: providing a short chain fatty acid ester of vinyl alcohol corresponding to a repeat unit of Formula I and a polymerizable unsaturated monomer, e.g., maleic anhydride or vinyl chloride, which carries a carboxy group or its protected group or a halogen atom (Cl, Br, etc.); radical polymerizing these substances in the presence of a radical initiator; metal-alcoholating (e.g., lithiating) a hydroxyl group at one end, for example, in an acid anhydride unit or vinyl chloride unit of the obtained random copolymer, or grafting a poly(ethylene glycol) chain via ester or amide bonds or ethers (-O-) or -NH- using a poly(ethylene glycol) derivative obtained by converting the hydroxyl group at the one end into an amino group ($NH_2$). When a chain transfer agent such as a cyanomethyl methyl(phenyl) carbamodithioate or cyanomethyl methyl(phenyl) carbamodithioate is used in combination during the polymerization reaction described above, a molecular weight of the synthesized polymer can be reduced, and an operation of a subsequent grafting reaction can be simplified. Of course, a copolymer of interest can also be produced without using such a chain transfer agent.

The nanoparticles or polymeric micelles described above are made by preparing an aqueous solution containing a water-soluble organic solvent, e.g., N,N-dimethylformamide (DMF) or dimethyl sulfoxide (DMSO), and then dialyzing the solution against water via a dialysis membrane with a constant molecular weight cut-off so that the copolymer according to the present invention, which is amphipathic, itself is associated to form micelles. The micelles or nanoparticles thus formed can be obtained as separated solids, for example, by freeze drying or centrifugation.

The nanoparticles and nano-sized polymeric micelles thus provided can be provided as a solubilized or homogeneously dispersed solution or liquid agent in an aqueous medium (if necessary, an aqueous solution that can contain a physiological salt or a pH modifier), and thus can be provided as oral formulations in various forms, including parenteral

formulations. For example, when provided as an oral formulation, the nanoparticle of the present invention can also be provided as a tablet, a pill, or a granule, by using an excipient or a diluent, which is per se commonly used in the art. The excipient or diluent can be, but is not limited to, chlorocarmellose sodium, crystalline cellulose, hypromellose, sodium lauryl sulfate, magnesium stearate, macrogol 4000, titanium oxide, or the like, which is commonly used in the art.

A pharmaceutical formulation containing such a nanoparticle as an active ingredient can exert physiological functions inherent in the short chain fatty acid itself as described above locally in a living body of a mammal, including a human, to which the nanoparticle is to be delivered. A dose of such a pharmaceutical formulation cannot be uniquely identified because an optimal dose varies depending on the disease to be treated or administration method, but can be determined by a specialist based on data and the like obtained through a small-scale clinical trial or the like.

Description of Embodiments

Hereinafter, typical examples of the present invention will be described in detail for the avoidance of complicated description, but the present invention is not limited to these examples.

Production Example 1: Synthesis of $CH_3O\text{-}(CH_2CH_2O)_m\text{-}CH_2PhCH_2Br$ (N686)

To commercially available $CH_3O\text{-}(CH_2CH_2O)_m\text{-}H$ (MW = 5,000, 100 g, 20 mmol), 200 mL of tetrahydrofuran (THF) and 14.4 mL of butyllithium (23 mmol, 1.6 M-hexanes) were added. Then, α,α'-dibromoxylene (25 g, 95 mmol) was added, and they were reacted at 50°C for 2 days. After precipitation of the reaction product in 2-propanol (IPA), the precipitate was dried under reduced pressure. The resulting pale yellow polymer was dissolved in methanol and centrifuged to remove α,α'-dibromoxylene as a precipitate. A methanol solution was charged into IPA, and the resulting white precipitate was vacuum dried to give a target product (N686) (yield: 104 g).

Production Example 2: Synthesis of $CH_3O\text{-}(CH_2CH_2O)_m\text{-}CH_2PhCH_2S(=S)OCH_2CH_3$ (N717)

N686 (20 g), which was synthesized in Production Example 1, was dissolved in 100 mL of ethanol, potassium ethylxanthate ($CH_3CH_2OC(=S)SK$, 7 g) was added, and they were reacted at room temperature for 10 minutes. After centrifugal fractionation of the precipitate, ethanol was distilled off under reduced pressure. The residue was dissolved in chloroform and washed with water, and the chloroform layer was fractionated. After anhydrous sodium sulfate dehydration and filtration, the product was precipitated in IPA. After centrifugation, the product was dried under reduced pressure to give a target product (N717) (yield: 15 g).

Production Example 3: Synthesis of $CH_3O\text{-}(CH_2CH_2O)_m\text{-}CH_2PhCH_2[CH_2CH(OC(=O)CH_3)]_nS(=S)OCH_2CH_3$ (N684)

N717 (1 g) synthesized in Production Example 2, azobisisobutyronitrile (15 mg), and vinyl acetate (6.6 g) were added to a flask, subjected to nitrogen bubbling for 5 minutes, and then reacted at 60°C for 1 day. The resulting target product was dissolved in THF, precipitated in IPA, and dried under reduced pressure to give a target product (N684) (2.6 g). A $^1H$ NMR spectrum of N684 is illustrated in FIG. 1.

Production Example 4: Synthesis of $CH_3O\text{-}(CH_2CH_2O)_m\text{-}CH_2PhCH_2[CH_2CH(OC(=O)CH_2CH_3)]_nS(=S)OCH_2CH_3$ (N731)

N717 (5 g) synthesized in Production Example 2, azobisisobutyronitrile (75 mg), and vinyl propionate (7 g) were added to a flask, subjected to nitrogen bubbling for 5 minutes, and then reacted at 60°C for 2 days. The resulting target product was dissolved in THF, precipitated in IPA, and dried under reduced pressure to give the target (9.3 g). A $^1H$ NMR spectrum of N731 is illustrated in FIG. 2.

Production Example 5: Synthesis of $CH_3O\text{-}(CH_2CH_2O)_m\text{-}CH_2PhCH_2[CH_2CH(OC(=O)CH_2CH_2CH_3)]_nS(=S)OCH_2CH_3$ (N721)

N717 (5 g) synthesized in Example 2, azobisisobutyronitrile (75 mg), and vinyl butyrate (10 g) were added to a flask, subjected to nitrogen bubbling for 5 minutes, and then reacted at 60°C for 2 days. The resulting target product was dissolved in THF, precipitated in IPA, and dried under reduced pressure to give a target product (N721) (12.3 g). A $^1H$ NMR spectrum of N721 is illustrated in FIG. 3.

Production Example 6: Synthesis of the graft copolymer (N741)

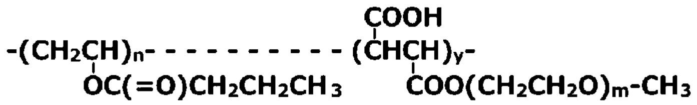

(N741)

Azobisisobutyronitrile (75 mg), vinyl butyrate (5.7 g), maleic anhydride (100 mg), and cyanomethyl methyl(phenyl) carbamodithioate, ($PhN(CH_3)C(=S)SCH_2CN$, 200 mg) were added to a flask, subjected to nitrogen bubbling for 5 minutes, and then reacted at 60°C for 2 days. The resulting target product was dissolved in THF, and after small-amount sampling, and a THF solution of $CH_3O\text{-}(CH_2CH_2O)_mOLi^{*Note}$ prepared separately was added and stirred 30 minutes. The reaction solution was poured into IPA, and the precipitate was dried to give a target product (N741) (6 g). A $^1H$ NMR spectrum of N741 is illustrated in FIG. 4.

*Note: to THF (20 mL), $CH_3O\text{-}(CH_2CH_2O)_mOH$ (MW = 5,000; 5 g) and butyl lithium (0.6 mL) were added to make a THF solution of $CH_3O\text{-}(CH_2CH_2O)_mOLi$.

Production Example 7: Preparation 1 of self-assembled particles

Fifty (50) mg of each polymer synthesized above (N684, N731, and N721) was collected and dissolved in 1 mL of DMF; 1 mL of water was added; and the solution was put in a dialysis membrane (MWCO = 3.5 KDa) and dialyzed against 2 L of water. After exchanging the dialysis water three times every half day, dynamic light scattering measurement was performed, and it was confirmed that particles having an average from 30 to 100 nm were formed (see FIG. 5).

Production Example 8: Preparation 2 of self-assembled particles

The operation of Production Example 7 was repeated except that the graft polymer (N741) synthesized in Production Example 6 was used, and it was confirmed that particles having an average particle size of 153 nm were formed (see FIG. 6).

Test Example 1: Cytotoxicity 1

To a 96-well plate seeded with $1 \times 10^4$ HepG2 cells, each sample of the self-assembled particles made in Production Example 7 was added, and incubated for 24 hours. Then, a WST solution was added, and, after 2 hours, UV absorption at 450 nm was measured and compared with that of a control. As a result, it was confirmed that all of the samples hardly exhibited toxicity at the measurement concentrations (Fig. 7 illustrates cytotoxicity of each of the nanoparticles N684, N731, and N721).

Test Example 2: Cytotoxicity 2

The operation of Test Example 1 was repeated except that the self-assembled particles made in Production Example 8 were used, and the cytotoxicity of N741 was evaluated. The test results are illustrated in FIG. 8. From FIG. 8, the nanoparticles of N741 are found not to be cytotoxic in this test system.

Test Example 3: Cancer metastasis suppressive effect

From five to seven 5-week-old BALB/c mice (male) were allowed to freely take each sample described in the following administration groups GP1 to GP6. Two days later, the mice were tail vein injected with $1 \times 10^4$ B16F10/B16F10 melanoma cells (obtained from RIKEN Cell Bank), and the intake of the sample water was continued. The mice were dissected on Day 11, and the number of cancers rightly moved to the lung were observed by the naked eye. The results of the observation are illustrated in FIG. 10, and the body weight changes of experimental animals during testing are illustrated in FIG. 9.

From FIG. 10, the short chain fatty acids showed almost no effect of suppressing the number of cancer metastases, whereas the GP5 group of propionic acid nanoparticles showed extremely high suppression of cancer metastasis. FIG. 11 illustrates the results of measuring, under a microscope, a number of micrometastases that cannot be observed by the naked eye. Similarly to FIG. 10, it was confirmed that propionic acid nanoparticles (abbreviated as PEG-b-PVPro in the figure) extremely suppressed cancer metastasis.

FIG. 12 shows H & E staining views (left) of lung tissue views and the number of metastatic cancers (right) determined from the views. A significant reduction in the number of cancers was observed with the propionic acid nanoparticles and the butyric acid nanoparticles as compared with the control. FIG. 13 shows H & E staining views (left) of lung tissue views and an area of metastatic cancer (right) determined from the views. A significant reduction in the area of cancer was observed with the propionic acid nanoparticles and the butyric acid nanoparticles as compared with the control and low molecular weight fatty acids. FIG. 14 shows ALT, AST, LDH and ALB levels in the blood. It was confirmed that the liver and organs were hardly impaired in any case.

FIGS. 15 to 18 show H & E staining views of the small intestine and colorectal tissue. It was confirmed that, in the low molecular weight fatty acid groups, villus shortening and damage were observed at some locations.

Sample administration group

GP1: Healthy group (n=5)
GP2: Melanoma administration group (n=7)
GP3: Melanoma administration group/30 mM propionic acid free intake group (n=7)
GP4: Melanoma administration group/30 mM butyric acid free intake group (n=7)
GP5: Melanoma administration group/30mM N731 free intake group (n=7)
GP6: Melanoma administration group/30mM N721 free intake group (n=7)

Test Example 4: Diet effect

Five 4-week-old C57BL/6J mice (male) per group were fed with a solid feed D12492 (60% Fat, ultra-high fat feed) purchased from EPS EKISHIN Co., Ltd. Each of the following administration groups GP1 to GP3 was fed with each sample described, and their body weight was measured. The results are illustrated in FIG. 19.

From FIG. 19, it was confirmed that the butyric acid particle group (N721) significantly suppressed the body weight increase as compared with the tap water group and the propionic acid particles (N731).

Sample administration group

GP1: Tap water free intake
GP2: N731 free intake (5 mg/mL)
GP3: N721 free intake (5 mg/mL)

Test Example 5: Effect on ulcerative colitis

Severn 7-week-old ICR mice (male) per group were allowed to freely take 4% sodium dextran sulfate (DSS), and orally administered with the following samples once daily with a probe. The disease activity index (DAI) was measured after 10 days, and the blood was evaluated. As illustrated in FIG. 20, the butyric acid nanoparticles (designated as BNP in the figure) gave a significant reduction in disease activity index in the ulcerative colitis model, and therapeutic effects were observed. In addition, as illustrated in FIG. 21, a significant increase in white blood cell count was observed in the ulcerative colitis model, whereas BNP significantly suppressed such an increase.

Sample administration group

GP1: Tap water free intake + tap water (0.65 mL)
GP2: 4% DSS intake + tap water (0.65 mL)
GP3: 4% DSS intake + butyric acid (2.32 mg/mL, 0.65 mL)
GP4: 4% DSS intake + CNP (PEG-b-polystyrene) (10 mg/mL, 0.65 mL)
GP5: 4% DSS intake + BNP (PEG-b-poly(viylburyrate) (10 mg/mL, 0.65 mL)

Test Example 6: Effect on non-alcoholic steatohepatitis (NASH)

Forty-nine (49) 5-week-old C57BL/6J mice (male) were allowed to freely take a solid feed A06071302 purchased from EPS EKISHIN Co., Ltd. (choline-deficient high-fat feed, methionine weight decreased, 0.1% methionine added), and randomly divided into groups each including 7 mice after 4 weeks. Each sample described was administered, through the free intake, to each of the following administration groups GP1 to GP7. Data analysis was performed after 8 weeks. FIG. 22 shows liver and spleen weights. It was confirmed that enlargement occurred due to inflammation in the NASH group, whereas the enlargement was significantly suppressed in the propionic acid particle administration group.

Sample administration group

GP1: Normal solid feed (Oriental Yeast MF)
GP2: Solid feed A06071302
GP3: Solid feed A06071302 + butyric acid (65 mM)
GP4: Solid feed A06071302 + propionic acid (50 mM)
GP5: Solid feed A06071302 + butyric acid nanoparticles (10 mg/mL, polymer concentration in terms of 1 mM butyric acid: 65 mM)
GP6: Solid feed A06071302 + propionic acid nanoparticles (10 mg/mL, polymer concentration in terms of 1 mM propionic acid: 50 mM)
GP7: Solid feed A06071302 + polystyrene nanoparticles (10 mg/mL)

Test Example 7: Cancer metastasis suppressive effect 2

Five to seven 7 to 8-week-old C57BL/6J mice (male) per group were obtained from Charles River Japan, Inc (Yokohama). These mice were bred while being freely fed with a standard solid feed at controlled temperature (23 $\pm$1°C) and humidity (50 $\pm$5%) in a controlled temperature (23 $\pm$1°C) and humidity (50 $\pm$5%) under non-pathogenic conditions in 12 hour dark/light cycles. The mice were randomly divided into the following administration groups GP1 to GP6 and allowed to freely take each sample described. One day later, the mice were tail vain injected with 2.5 $\times$ $10^5$ B16F10/B16F10 melanoma cells per 200 $\mu$L (saline) (obtained from RIKEN Cell Bank). Each sample was given to the mice in a free water drinking manner from 1 day before this tail vain injection to the end point of the test, Day 11. On Day 11, the plasma and other organs were collected and stored appropriately for each further analysis which will be described below.

Changes in consumptions of the samples under test and body weight changes of experimental animals are illustrated in FIG. 23. Other tests yielded test results that were substantially equivalent to the test results observed or confirmed in Test Example 3.

Sample administration group

GP1: Healthy group (n=5)
GP2: Melanoma administration group (n=7)
GP3: Melanoma administration group/30 mM propionic acid free intake group (n=7)
GP4: Melanoma administration group/30 mM butyric acid free intake group (n=7)
GP5: Melanoma administration group/30 mM PNP (N731-derived particles: see Production Example 7) free intake group (n=7)
GP6: Melanoma administration group/30 mM BNP (N721-derived particles: see Production Example 7) free intake group (n=7)

Test Example 8: Effect on antidiabetic test

The management and use of animals in this test and in the tests using all experimental animals disclosed in the present specification were performed strictly in accordance with the guidelines of the University of Tsukuba regarding the management and the like.

Seven 7 to 8-week-old C57BL/6J mice (male) per group were obtained from Charles River Japan, Inc (Yokohama). These mice were bred while being freely fed with a standard solid feed at controlled temperature (23 $\pm$1°C) and

humidity (50 ±5%) in a controlled temperature (23 ±1°C) and humidity (50 ±5%) under non-pathogenic conditions in 12 hours dark/light cycles. The mice were randomly divided into the following administration groups of GP1 to GP6, and allowed to freely drink each sample described until Day 36. One day later, a glucose resistance test was performed. In the test, the body weight and sample consumption for each mouse were monitored every day. The sample was then replaced with drinking water until the end of the test (Day 40).

Sample administration group

GP1: Exenatide (traditional antidiabetic agent), 1 μg (Days 1 to 4) or 2 μg (Days 5 to 36) daily subcutaneous injection group
GP2: 60 mM BNP (N721-derived particles: see Production Example 7) free intake group
GP3: 60 mM PNP (N721-derived particles: see Production Example 7) free intake group
GP4: 30 mM butyric acid free intake group
GP5: 30 mM propionic acid free intake group
GP6: Control group (water free intake)

(1) In FIG. 24, the sample consumption in the test is indicated as volume (mL) per mouse, and the mouse body weight is indicated as mean ± SD values.
(2) The glucose resistance test was performed to evaluate the therapeutic effects of short chain fatty acids in controlling the metabolism of the administered glucose. Sixteen (16) hours after overnight constraint, glucose (2 g/kg) was orally administered to the mice. One hour before and after the glucose administration, 10 μL of blood was collected from the tail vein, and mixed with heparin-containing (50 units/mL) saline in a volume (v: v) ratio of 1:1. A glucose concentration in the diluted blood was measured by FUJI DRY-CHEM 7000V (FUJIFILM Corporation). A final blood glucose concentration was calculated by the following equation:

$$\text{Final glucose concentration (mg/mL)} = [\text{glucose}]_{60\ \text{min}} - [\text{glucose}]_{0\ \text{min}}$$

The data as mean ± SEM (n=7) values are illustrated in FIG. 25.
Student's t-test, tail (2) and type (2), was performed to determine a statistical error between the average values (P < 0.05 was considered statistically significant).
As compared with the diabetic model mice, the propionic acid administration group, the butyric acid administration group, and the propionic acid nanoparticle (PNP) administration group showed no significant differences, while the glucose concentration could be significantly reduced in the diabetic drug exenatide and BNP administration groups, demonstrating that the pancreatic function was improved.

(3) Organ weights at end of the test

Results of measuring weights of the spleen, kidney, and liver were taken out rapidly after dissection of the mice were illustrated in FIG. 26. These organs were stored in a 10% neutral buffer for further histological analysis. The Student's t-test is the same as described above.
In the results, the spleen weight was increased in the propionic acid administration group, and the liver weight increase was confirmed in the butyric acid administration group and the exenatide administration group. This indicates organ inflammation. On the other hand, the BNP administration group is comparable to the control all in terms of spleen, kidney, and liver, and BNP is not toxic.

(4) Histological analysis of H & E stained intestinal tract

The organs of the mice taken out after dissection were fixed by immediately putting them in a 10% neutral formalin solution and immersing them therein for 1 day. Thereafter, the solution was replaced with a 70% ethanol solution for paraffin embedding. All the organs after the paraffin embedding were processed into tissue sections with a thickness of 5 μm, which were hematoxylin/eosin (HE) stained by a routine method. The tissue sections were dehydrated with high-concentration alcohol and washed with xylene, followed by microscope examination (biorevo, BZ-9000, Keyence). A villus length was measured by ImageJ software (NIH). The results are illustrated in FIG. 29. As quantitative data,

minimum and maximum range values, upper and lower quartiles, and median: duodenum (n = from 130 to 132), jejunum (n = from 96 to 185), ileum (n = from 109 to 138), and colon (n = from 37 to 47) were displayed as a plot in the box. The t-test is the same as described above.

It is indicated that the exenatide administration group had a significantly shortened villus length in the duodenum, jejunum, ileum, and large intestine, and exhibited strong side effects. The propionic acid and butyric acid administration groups are also in the same trend. On the other hand, it can be seen that the BNP administration group and the PNP administration group did not have a shortened villus length or damage to the digestive tract.

(5) Histological analysis of H & E stained pancreatic tissue

The organs of the mice taken out after dissection were fixed by immediately putting them in a 10% neutral formalin solution and immersing them therein for 1 day. Thereafter, the solution was replaced with a 70% ethanol solution for paraffin embedding. In accordance with a routine method, all the organs after the paraffin embedding were processed into tissue sections with a thickness of 5 $\mu$m, which were hematoxylin/eosin (HE) stained by a routine method. The tissue sections were dehydrated with high-concentration alcohol, washed with xylene, then fixed, and subjected to microscope examination (biorevo, BZ-9000, Keyence). A region of the islets of Langerhans was determined by ImageJ software (NIH). The results are illustrated in FIG. 30. As quantitative data, minimum and maximum range values, upper and lower quartiles, and median (n = from 10 to 20) were displayed as a plot in the box. The t-test is the same as described above.

It is indicated that, in the diabetic model mice, and the propionic acid administration group, the butyric acid administration group and the PNP administration group, the size of the islets of Langerhans was reduced, but the BNP administration group and the exenatide administration group did not show atrophy of the islets of Langerhans and maintained the function of the islets of Langerhans.

Test Example 9: Effect 1 as radiation enhancer

Five 5 to 7-week-old C57BL/6J mice per group obtained similarly as in the above test were bred under similar conditions. $0.076 \times 10^6$ melanoma B16F10 cells per 100 $\mu$L (serum free DMEM) were injected subcutaneously into the outer side of the right thigh of each of the mice (Day 7 before irradiation). After 1 week of cancer proliferation, the mice were randomly divided into the following administration groups. BNP (500 mg/kg) was administered intraperitoneally (i.p.) to the mice 1 day before irradiation and immediately after irradiation to confirm a radiation enhancement effect (GP3 and GP4, respectively). The irradiation conditions were set to 10 Gy, 150 kV, 20 mA, Al filtration, and 330 mm intervals.

Sample administration group

GP1: Cancer control group
GP2: Cancer + irradiation (IR: 10 Gy) group
GP3: BNP 500 mg/kg (Day 1 before irradiation) group
GP4: BNP 500 mg/kg (Day 0 after irradiation) group

(1) After the irradiation, the cancer proliferation and body weight were followed until the end of the test (8 days). The results are illustrated in FIG. 31. Day 0 is data before the irradiation, and Day 8 is data after the irradiation (at the end of the test).

No body weight change or toxicity is observed in the BNP administration group, similarly to the non-administration group.

(2) Plasma and other organs were collected at the end of the test and stored properly for further analysis. A cancer size was measured with a caliper, and a cancer volume was calculated using the following equation:

$$\text{Cancer volume} = 0.52 \text{ x length x width}^2$$

The results are illustrated in FIG. 30. The t-test is the same as in Test 8 (2).

A tumor growth suppressive effect was observed in the 10 Gy irradiation group, and, even when BNP was administered before and after X-ray irradiation, the tumor size after 8 days was significantly smaller than that in the BNP non-administration group, confirming the radiation enhancement effect.

Test Example 10: Effect 2 as radiation enhancer

Five or seven 5 to 7-week-old C57BL/6J mice per group obtained similarly as in the above test were bred under similar conditions. 0.076 × $10^6$ melanoma B16F10 cells per 100 μL (serum free DMEM) were injected subcutaneously into the outer side of the right thigh of each of the mice (Day 9 before irradiation). When the cancer volume reached from 350 to 560 $mm^3$, the mice were randomly divided into the following administration groups. The irradiation conditions were set to 10 Gy, 150 kV, 20 mA, Al + Cu (0.5 mm + 0.1 mm) filtration, and 330 mm intervals.
The cancer volume was calculated in the same manner as described above.

Sample administration group

GP1: Healthy (saline) group (n=5)
GP2: Cancer control group (n=7)
GP3: Cancer + irradiation (IR (5Gy)) group (n=7)
GP4: Butyric acid (i.p.; 250 mg/kg) (6 hours before irradiation) + IR group (n=7)
GP5: Butyric acid (i.p.; 500 mg/kg) (24 hours before irradiation) + IR group (n=7)
GP6: BNP (i.p.: 500 mg/kg) (6 hours before irradiation) + IR group (n=7)
GP7: BNP (i.p.: 500 mg/kg) (12 hours before irradiation) + IR group (n=7)

(1) The data on cancer proliferation profiles by 5 Gy irradiation after butyric acid and NP administration in a melanoma xenograft model is illustrated in FIG. 33.
Butyric acid and BNP were administered prior to radiation irradiation. The tumor proliferation suppressive effect increased in the order: irradiation group < butyric acid (6 hours before) administration group < BNP (6 hours before) administration group = butyric acid (24 hours before) administration group < BNP (24 hours before) administration group.
(2) The data on the cancer weight at the end of the test (Day 6 after irradiation) is illustrated in FIG. 32 as mean ± SEM values (n = from 2 to 7). The t-test is the same as in Test 8 (5).
According to the tumor weight results, the tumor size was significantly reduced in the butyric acid (24 hours before) administration group and the butyric acid (6 hours before) administration group. (3) The data on body weight changes in the mice as mean ± SD values (n = 7) are illustrated in FIG. 35. The t-test is the same as described above.
A body weight decrease is found in butyrate administration groups, and toxicity is developed. On the other hand, the BNP administration group does not have a body weight decrease, and toxicity is not developed.

Test Example 11: In vitro evaluation of radiation enhancement effect of BNP evaluated by spheroid test

Melanoma B16F10 cells were treated with 50 mM butyric acid for 7 hours and then irradiated with 2 Gy (Al + Cu (0.5 mm + 0.1 mm) filter). The irradiation was kept for 1 hour at 37°C, the medium was removed, and the sample was removed through several times of washing. Then, a fresh medium was added. The treated cells were incubated for 24 hours and then cultured in 0.6% methylcellulose and DMEM (1: 1) for 3 days to form spheroids. Images were taken by direct microscopy and the spheroids were measured by ImageJ software (NIH). The cells were grouped and then treated with samples for 7 hours after 2 Gy irradiation. The data are displayed as mean ± SEM (n = from 32 to 37) values. These results are illustrated in FIG. 38. The t-test is the same as described above.

Cell culture:

Mouse melanoma B16F10 cells were purchased from CellBankk (RIKEN, Japan). These cell lines were held at 37°C under a humid atmosphere of 5% $CO_2$ in a Dulbecco's modified Eagle's medium (DMEM; L-glutamine, 1 g/L glucose, sodium bicarbonate, Sigma-Aldrich, St. Louis, MO, USA) supplemented with 10% fetal bovine serum and 100 ng/mL of a penicillin-streptomycin-neomycin antibiotic mixture. The test results are illustrated in FIG. 34.
A spheroid cancer cell growth suppressive effect is confirmed clearly in the BNP administration group.

Test Example 12: Effect 2 on non-alcoholic steatohepatitis (NASH)

Forty-two (42) 5-week-old C57BL/6J mice (male) were allowed to freely take a solid feed A06071302 purchased from EPS EKISHIN Co., Ltd. (choline-deficient high-fat feed, methionine weight decreased, 0.1% methionine added), and randomly divided into groups each including 7 mice after 4 weeks. Each sample described was administered, through the free intake, to each of the following six groups: healthy group (Healthy) (solid feed A06071302 not administered),

NASH, butyric acid administration (BA), propionic acid administration (PA), PNP administration, and BNP administration (each sample was prepared to attain a PA concentration of 65 mM for the PA and PNP groups, and prepared to attain a BA concentration of 50 mM for the BA and BNP groups). Data analysis was performed after 8 weeks.

(1) Liver and spleen weights are illustrated in FIG. 35. It was confirmed that enlargement occurred due to inflammation in the NASH group, whereas the enlargement was significantly suppressed in the propionic acid particle administration group.

Here, in order to compare the difference in the amount of fat accumulated in the liver and the degree of swelling in response to inflammation in the liver, the weights of the liver and spleen taken out after dissection of the mice were measured, and average weights of each group were calculated (each group n = 7). As error bars of the average liver weight value and the average spleen weight value of each group, a standard deviation value (n = 7 for each group) was used. The t-test is performed to examine the presence or absence of statistical significance between average values, and $P < 0.05$ indicates a statistically significant difference.

As for the liver weight (left figure), the liver weight of the NASH group is significantly heavy relative to the liver weight of the healthy group, and inflammation is caused. On the other hand, the PNP administration group is significantly lighter in liver weight than the NASH group, and inflammation is suppressed.

Similarly, as for the spleen weight (left figure), the spleen weight of the NASH group significantly increases relative to that of the healthy group, and the spleen weight of the PNP administration group significantly decreases.

(2) Histological analysis of HE stained liver

The liver of the mice taken out after dissection was fixed by immediately putting it in a 10% neutral buffer formalin solution and immersing it therein for 1 day. Thereafter, the solution was replaced with a 70% ethanol solution for paraffin embedding. All the livers after the paraffin embedding were processed into tissue sections with a thickness of 5 μm, which were hematoxylin/eosin (HE) stained. All the tissue sections after staining were imaged by a microscope (all-in-one fluorescence microscope, BZ-X710, Keyence), and then a proportion of oil droplet tissue area relative to liver tissue area by ImageJ (NIH) (each group n = 7). The measurement results are illustrated in FIG. 36. The graph shows an error bar based on the average value and standard deviation of the oil droplet tissue area of each group. The t-test is performed to examine the presence or absence of a statistical significance between average values, and $P < 0.05$ indicates statistically significant difference.

The oil droplet volume of the liver of the NASH group significantly increases relative to that of the healthy group (fatty liver condition). On the other hand, the PNP administration group has significantly less oil droplet volume as compared with the NASH group.

(3) Histological analysis of MT stained liver

The liver of the mice taken out after dissection was fixed by immediately putting it in a 10% neutral buffer formalin solution and immersing it therein for 1 day. Thereafter, the solution was replaced with a 70% ethanol solution for paraffin embedding. All the livers after the paraffin embedding were processed into tissue sections with a thickness of 5 μm, which were Masson trichrome (MT) stained. All the tissue sections after staining were imaged by a microscope (all-in-one fluorescence microscope, BZ-X710, Keyence), and then a proportion of fibrotic tissue area relative to liver tissue area by ImageJ (NIH) (each group n = 7). The measurement results are illustrated in FIG. 42. The graph shows an error bar based on the average value and standard deviation of the fibrotic tissue area of each group. The T-test is performed to examine the presence or absence of statistical significance between average values, and $p < 0.05$ indicates a statistically significant difference.

The amount of liver fibrosis is significantly larger in the NASH group than in the healthy group. On the other hand, the amount of liver fibrosis is significantly smaller in the PNP administration group than in the NASH group.

Production Example 9: Synthesis of $CH_3O-(CH_2CH_2O)_m-CH_2PhCH_2[CH_2CH_2(OC(C=O)CH_2CH_2CH_2Ph)_n]S(=S)OCH_2CH_3$ (N821)

N817 (1.5 g) synthesized in the same manner as for N717 synthesized in Production Example 2 (the present application), azobisisobutyronitrile (45 mg), and 4-phenylbutyric acid vinyl (2 g) were added to a flask, subjected to nitrogen bubbling for 5 minutes, and then reacted at 60°C for 1 day. The resulting target product was dissolved in THF, precipitated in isopropyl alcohol (IPA), and dried under reduced pressure to give a target product (N821) (2.8 g). $^1H$ NMR spectrum of N821 is shown in FIG. 38.

Production Example 10: Preparation of self-assembled particles (Ph-BNP)

Fifty (50) mg of the polymer (N821) synthesized in Production Example 9 was each collected and dissolved in 1 mL of DMF; 1 mL of water was added; and the solution was put in a dialysis membrane (MWCO = 3.5 KDa) and dialyzed against 2 L of water. After exchanging the dialysis water three times every half day, dynamic light scattering measurement was performed, and it could be confirmed that particles having an average of 67 nm were formed. The size distribution of PEG-b-poly(vinyl 4-phenylbutyric acid) nanoparticles (Ph-BNP, N832), which is a result of the dynamic scattering measurement, is illustrated in FIG. 44.

Test Example 13: Anti-ammonemia effect

Twenty-four (24) 6-week-old C57BL/6N mice (male) were randomly divided into 4 groups and samples were orally administered (once a day, 1.22 mmol-4 PBA/kg) thereto with a probe over 4 days. In the administration groups 2GP to 4GP, acetaminophen (acetyl-p-aminophenol; APAP; 300 mg/kg) was intraperitoneally administered on Day 4, causing acute liver disorder and hyperammonemia. The mice were dissected on Day 5, and the evaluation was performed.

<u>Sample administration group:</u>

1GP: Saline
2GP: APAP + water
3GP: APAP + 4-phenylbutyric acid (200 mg/kg, 1.22 mmol-4 PBA/kg)
4GP: APAP + Ph-BNP (200 mg/kg, 1.22 mmol-4PBA/kg)

(1) Biochemical assay of blood

Immediately after cardiac blood collection on Day 5 of the test, blood ammonia concentration, and plasma alanine aminotransferase (AST) and plasma alanine aminotransferase (ALT) levels as liver function indexes were each measured with an animal biochemical automated analyzer using a colorimetric slide. The results (oral administration effect of Ph-BNP on APAP acute liver disorder model) are illustrated in FIG. 40.

It can be seen, from the figure, that 4-Ph-BNP reduces the blood ammonia concentration and also significantly decreases the AST and ALT levels, and contributes to the liver function recovery.

(2) Histological analysis of HE stained liver

The liver of the mice taken out after dissection was stained according to the HE staining method for the organs or organs previously described. The results are illustrated in FIG. 41. It can be seen, from the figure, that the liver is strongly impaired by APAP administration, but that damage is suppressed by Ph-BNP. In the figure, Healthy corresponds to the healthy group mice; APAP administration group mice, APAP + PBA corresponds to the administration group of APAP and 4-phenylbutyric acid; APAP + Ph-BNP corresponds to the administration group of APAP and Ph-BNP (nanoparticles according to the present invention).

Test Example 15: Pharmacokinetic study

In accordance with the methods of Production Examples 1 to 8 described above, the modifications involved were performed according to the conventional methods. $^{125}$I was introduced into phenyl groups of PEG-b-poly(vinyl butyric acid) (see the left formula below) and PEG-b-poly(vinyl 4-phenylbutyric acid) (see the right formula below) having a benzene ring at an $\alpha$-terminal of PEG by a chloramine method, and they were purified twice on a PD-10 column to fractionate unreacted iodine.

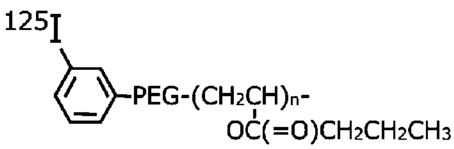

(N932)

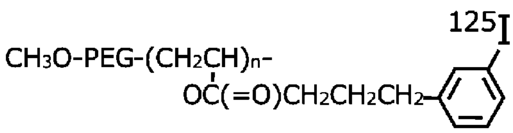

(N930)

After 24 hours, the blood, liver and digestive tract were collected from the mice which had been allowed to freely take N932 from the water bottle, and the gamma-ray intensity thereof was measured by a scintillation detector. The results are illustrated in FIG. 42 (left). In the figure, N932 was confirmed to be nearly completely localized to the digestive tract.

On the other hand, N930 was forcibly administered orally with a probe, and gamma intensities of the main organs were measured after 24 hours. The results are illustrated in FIG. 42 (right). From this figure, it can be confirmed that N930 is widely distributed in the blood, liver, kidney, etc. in addition to the digestive tract, and that 4-phenylbutyric acid is hydrolyzed in the digestive tract and taken up into the circulatory system.

Forty-five (45) 7-week-old ICR mice (male) were randomly divided into the following three groups. Doses for the administration groups 2GP and 3GP were determined so that the amount of phenylbutyric acid contained therein reached 200 mg/kg (1.22 mmol-4PBA/kg). After each sample is administered nearly simultaneously to all the mice, 1 mL of blood is taken by cardiac puncture, according to each endpoint (7 endpoints of 30 min, 1 h, 2 h, 4 h, 12 h, 16 h, 24 h), to separate plasma. The target organ, liver, was also taken out from each of the mice, and content of the phenyl butyric acid monomer was quantified by high performance liquid chromatography/mass spectrometry (LC/MS). The measurement results are illustrated in FIG. 43. In these figures, PBA was totally metabolized within 4 hours, whereas 4-Ph-BNP persistently released the phenylbutyric acid monomer in the blood and in the liver, and the release was not terminated even after 24 hours. Also, when the area under the concentration-time curve (AUC) was calculated, 4-Ph-BNP showed an approximately 3-time increase in the blood and approximately 15-time increase in the liver, as compared with PBA.

<u>Sample administration group:</u>

1GP: Saline (control, 3 mice)
2GP: 4-Phenylbutyric acid (7 endpoints (30 min, 1 h, 2 h, 4 h, 12 h, 16 h, and 24 h) x 3 mice)
3GP: Ph-BNP (7 endpoints (30 min, 1 h, 2 h, 4 h, 12 h, 16 h, and 24 h) x 3 mice)

## Claims

**1.** A hydrophilic-hydrophobic copolymer comprising:

(1) a hydrophobic segment derived from a repeat unit represented by Formula I:

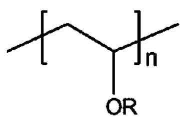

(I)

where in Formula I, R is -(C=O)$R^1$ or a hydrogen atom, in which $R^1$ is an unsubstituted or substituted linear or branched alkyl having from 1 to 7 carbon atoms (when $R^1$ is substituted, a substituent is unsubstituted or substituted phenyl, and a substituent of the substituted phenyl is one or more halogen, hydroxyl, or methyloxy), and the hydrogen atom, if present, exists in such a number that is 30% or less of n, where n is an integer from 5 to 1000; and
(2) a hydrophilic segment including a poly(ethylene glycol) chain that is:

(i) represented by Formula IIa:

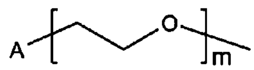

(IIa)

where in Formula IIa, A is unsubstituted or substituted $C_1$-$C_{12}$ alkyloxy, and when A is substituted, a substituent is a formyl group, formula R'R"CH- group, or a phenylamino group or phenethyl amino group, where R' and R" are independently $C_1$-$C_4$ alkyloxy, or R' and R" are taken together to form - $OCH_2CH_2O$-, -$O(CH_2)_3O$- or -$O(CH_2)_4O$-, and m is an integer from 10 to 500; or

(ii) derived from a repeat unit represented by Formula IIb:

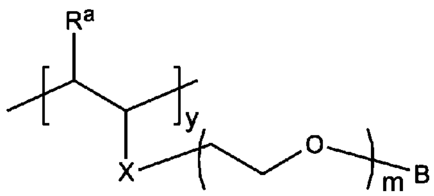

(I I b)

where in Formula IIb, $R^a$ is a hydrogen atom or a carboxy group,

X is C (=O)O or C(=O)NH when $R^a$ is a carboxy group, or is O or NH when $R^a$ is a hydrogen atom,
B is A-$CH_2CH_2$, and A and m are each as defined above, and
y is an integer from 1 to 300,

wherein the (1) hydrophobic segment and the (2) hydrophilic segment of (i) are each presented as blocks, and wherein each member of the repeat units including the (1) hydrophobic segment and the (2) hydrophilic segment of (ii) exists randomly with each other.

**2.** The hydrophilic-hydrophobic copolymer according to claim 1, which is associated and self-assembled in water to form a nanoparticle or a nano-sized polymeric micelle.

**3.** The hydrophilic-hydrophobic copolymer according to claim 1 or 2,
wherein the copolymer comprising the (1) hydrophobic segment derived from a repeat unit represented by Formula I and the (2) hydrophilic segment of (i) Formula IIa is a block copolymer represented by Formula BC:

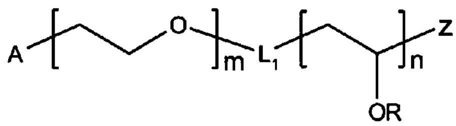

(B C)

where in Formula BC, A, R, m, and n are each as defined above, $L_1$ represents a direct bond or a divalent linking group, Z is a hydrogen atom, SH, S (C=S)-Ph, S(=S)$OCH_2CH_3$, a hydroxyl group, a $C_1$-$C_6$ alkyloxy group or an aryl-$C_1$-$C_2$ alkyloxy group, and
wherein the copolymer comprising the (1) hydrophobic segment derived from a repeat unit represented by Formula I and the (2) hydrophilic segment of (ii) Formula IIb is a graft copolymer represented by Formula RC:

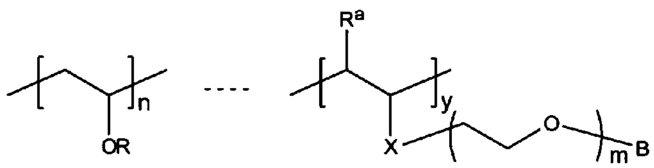

(G C)

where in Formula GC, R, $R^a$, B, X, m, n, and y are each as defined above.

**4.** A nanoparticle formed, in an aqueous medium, of the hydrophilic-hydrophobic copolymer according to any one of

claims 1 to 3.

5. A pharmaceutical formulation comprising, as an active ingredient, the hydrophilic-hydrophobic copolymer according to any one of claims 1 to 3 or the nanoparticle described in claim 4.

6. The pharmaceutical formulation according to claim 5 for use in preventing or treating cancer, suppressing obesity, preventing or treating ulcerative colitis or non-alcoholic fatty liver (or suppressing liver fibrosis), preventing or treating diabetes, enhancing radiation in radiation therapy, or preventing or treating hyperammonemia.

7. The hydrophilic-hydrophobic copolymer according to any one of claims 1 to 3 for use in preventing or treating cancer, suppressing obesity, preventing or treating ulcerative colitis or non-alcoholic fatty liver, preventing or treating diabetes, enhancing radiation in radiation therapy, or preventing or treating hyperammonemia.

8. The nanoparticle according to claim 4 for use in preventing or treating cancer, suppressing obesity, preventing or treating ulcerative colitis or non-alcoholic fatty liver (or suppressing liver fibrosis), preventing or treating diabetes, enhancing radiation in radiation therapy, or preventing or treating hyperammonemia.

9. A method for preventing or treating cancer, suppressing obesity, preventing or treating ulcerative colitis or non-alcoholic fatty liver (or suppressing liver fibrosis), preventing or treating diabetes, enhancing radiation in radiation therapy, or preventing or treating hyperammonemia, the method comprising administering the hydrophilic-hydrophobic copolymer according to any one of claims 1 to 3 to a patient in need thereof.

10. A method for preventing or treating cancer, suppressing obesity, preventing or treating ulcerative colitis or non-alcoholic fatty liver (or suppressing liver fibrosis), preventing or treating diabetes, enhancing radiation in radiation therapy, or preventing or treating hyperammonemia, the method comprising administering the nanoparticle according to claim 4 to a patient in need thereof.

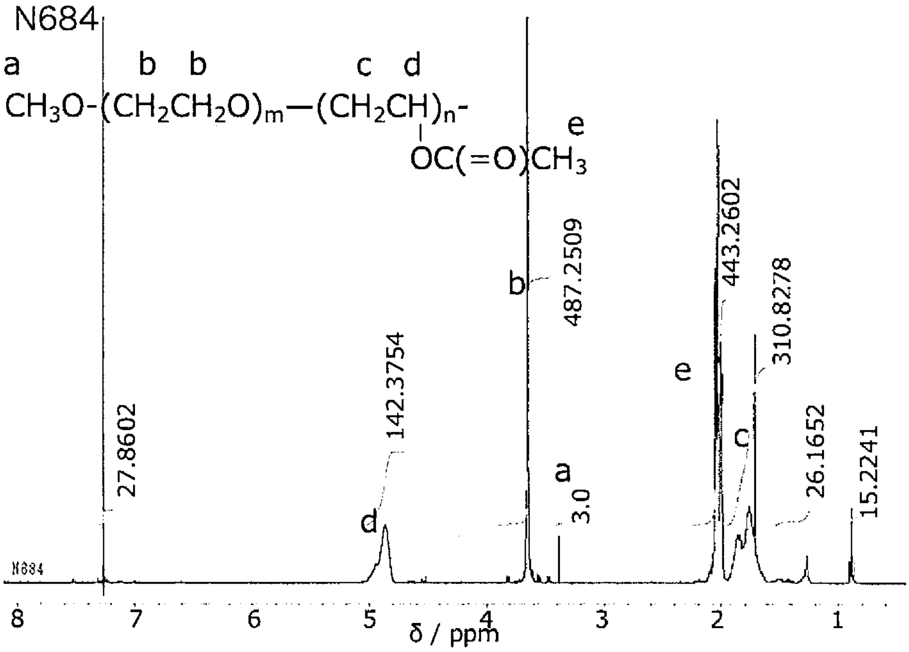
N684
a b b c d
CH3O-(CH2CH2O)m—(CH2CH)n-
e
OC(=O)CH3
487.2509
b
443.2602
e
310.8278
c
26.1652
15.2241
142.3754
d
27.8602
a
3.0
N684
8 7 6 5 4 3 2 1
δ / ppm

FIG. 1

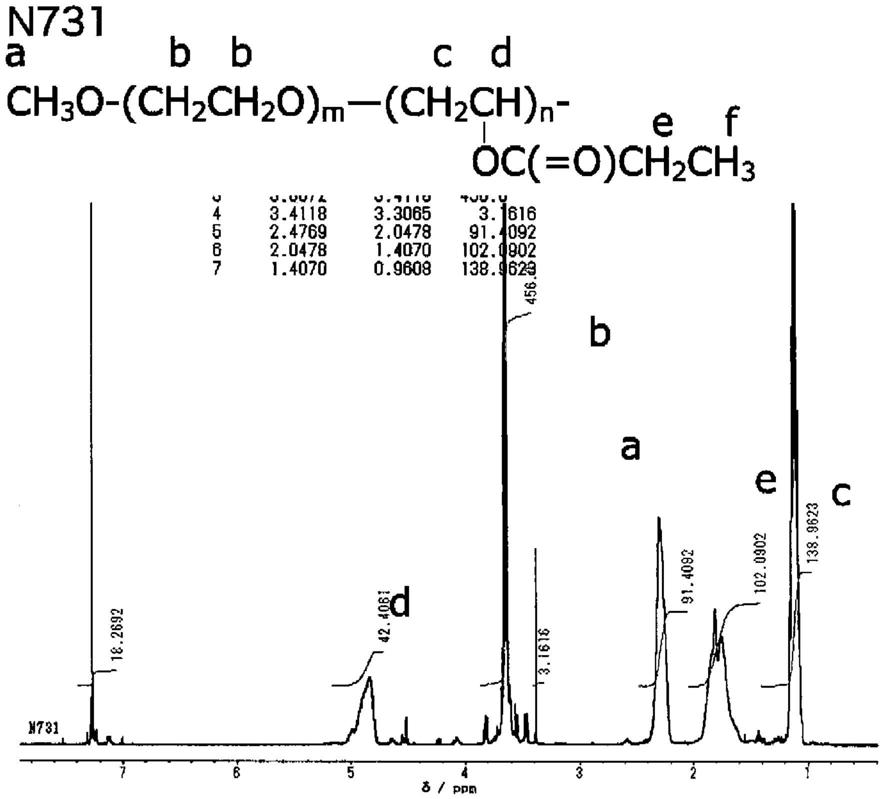
N731
a b b c d
CH3O-(CH2CH2O)m—(CH2CH)n-
e f
OC(=O)CH2CH3
4 3.4118 3.3065 3.1616
5 2.4769 2.0478 91.4092
6 2.0478 1.4070 102.0902
7 1.4070 0.9608 138.9623
b
a
e
c
d
18.2692
42.4061
3.1616
91.4092
102.0902
138.9623
N731
δ / ppm

FIG. 2

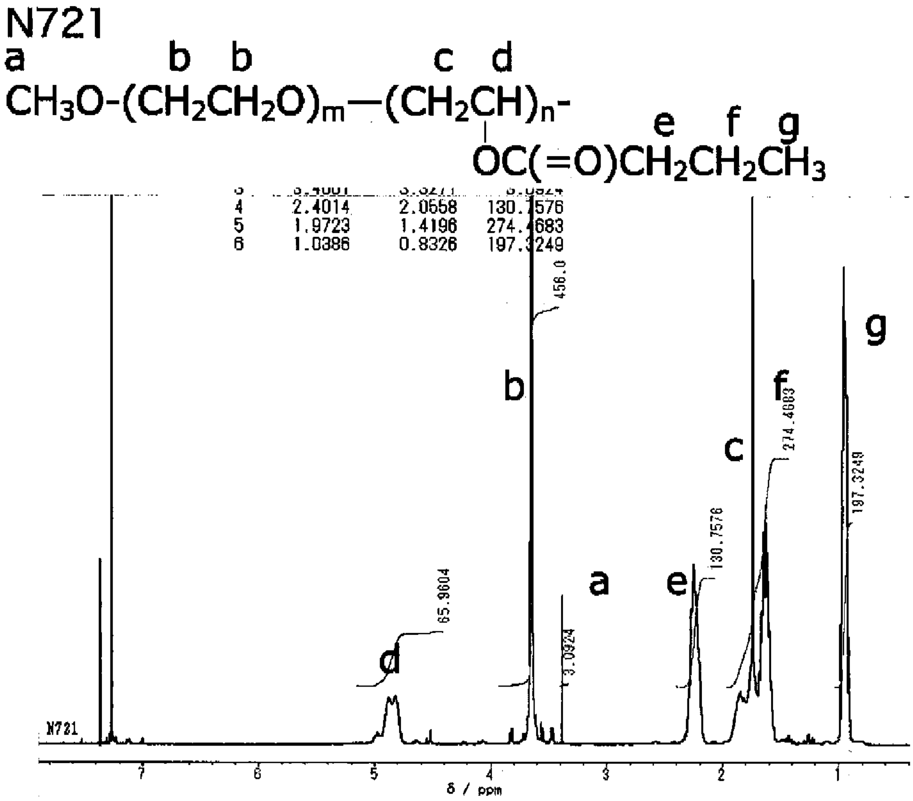
N721
a b b c d e f g
CH3O-(CH2CH2O)m—(CH2CH)n-
OC(=O)CH2CH2CH3
4 2.4014 2.0658 130.7576
5 1.9723 1.4196 274.4683
6 1.0386 0.8326 197.3249
456.0
b
g
f
c
274.4683
197.3249
130.7576
a
e
65.9604
d
3.0924
N721
7
6
5
4
3
2
1
δ / ppm

FIG. 3

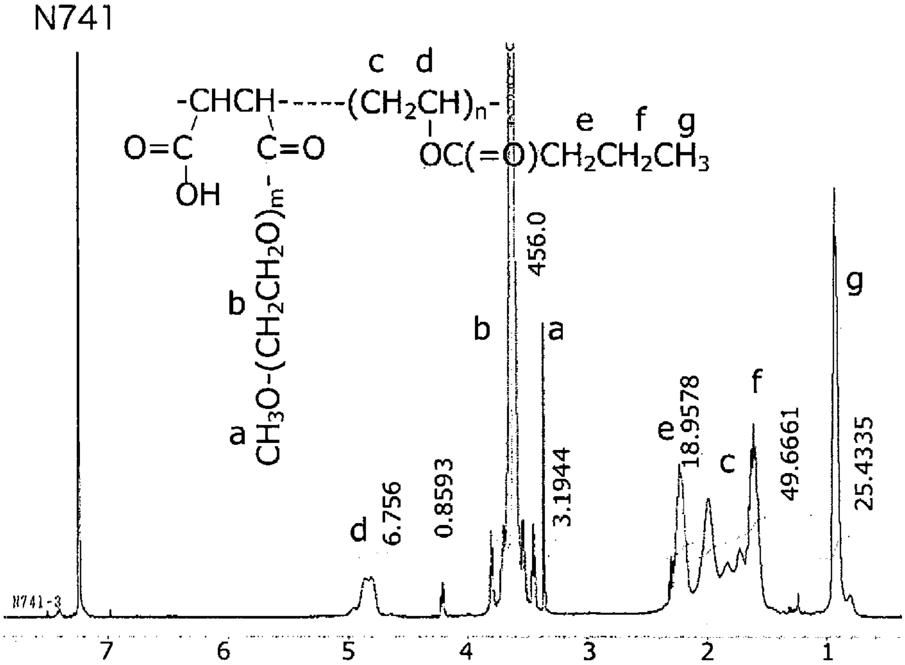
N741
456.0
6.756
0.8593
3.1944
18.9578
49.6661
25.4335
N741-3
7
6
5
4
3
2
1

FIG. 4

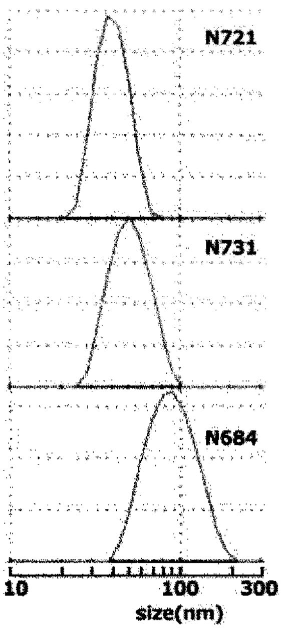


FIG. 5

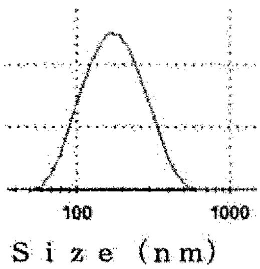


FIG. 6

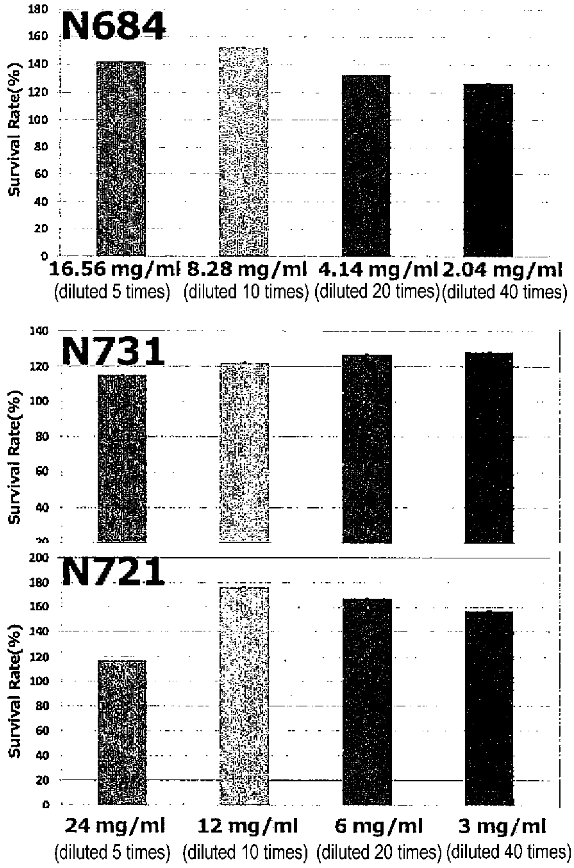
N684
Survival Rate(%)
16.56 mg/ml (diluted 5 times)
8.28 mg/ml (diluted 10 times)
4.14 mg/ml (diluted 20 times)
2.04 mg/ml (diluted 40 times)
N731
Survival Rate(%)
N721
Survival Rate(%)
24 mg/ml (diluted 5 times)
12 mg/ml (diluted 10 times)
6 mg/ml (diluted 20 times)
3 mg/ml (diluted 40 times)

FIG. 7

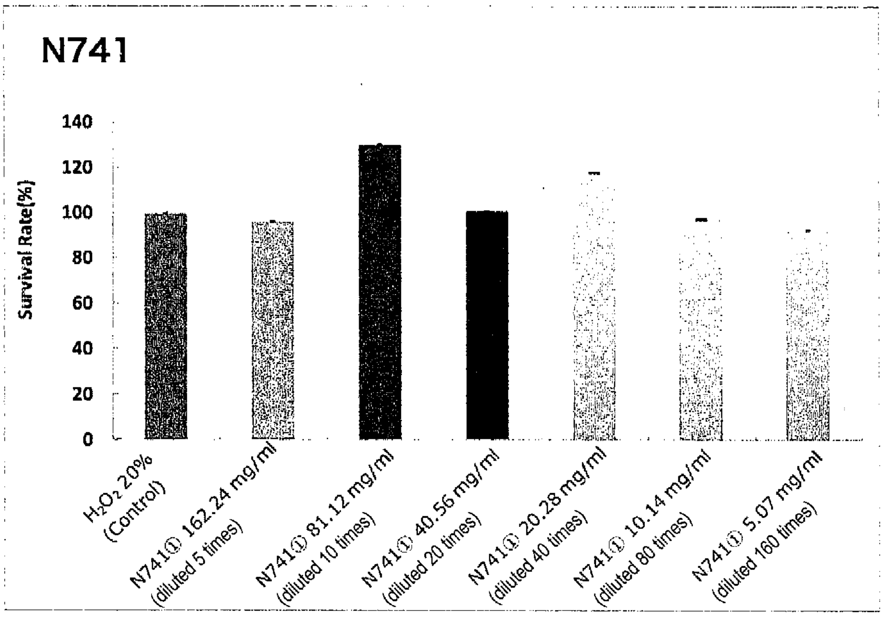
N741
140
120
100
80
60
40
20
0
Survival Rate(%)
$H_2O_2$ 20% (Control)
N741① 162.24 mg/ml (diluted 5 times)
N741① 81.12 mg/ml (diluted 10 times)
N741① 40.56 mg/ml (diluted 20 times)
N741① 20.28 mg/ml (diluted 40 times)
N741① 10.14 mg/ml (diluted 80 times)
N741① 5.07 mg/ml (diluted 160 times)

FIG. 8

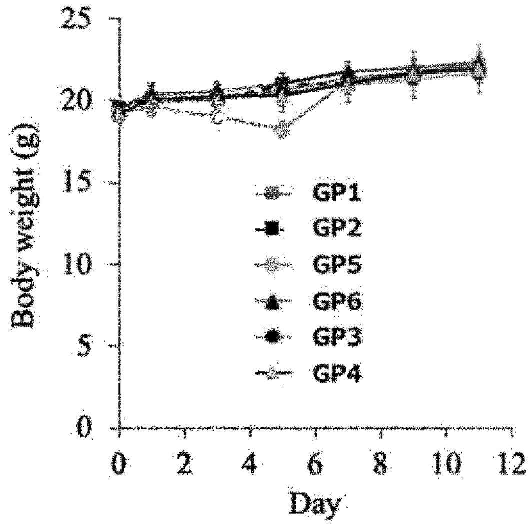

25
20
15
10
5
0
Body weight (g)
GP1
GP2
GP5
GP6
GP3
GP4
0
2
4
6
8
10
12
Day


FIG. 9

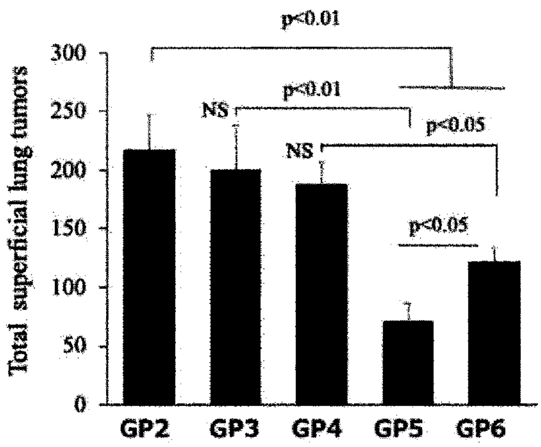
p<0.01
p<0.01
p<0.05
p<0.05
NS
NS
300
250
200
150
100
50
0
Total superficial lung tumors
GP2
GP3
GP4
GP5
GP6

FIG. 10

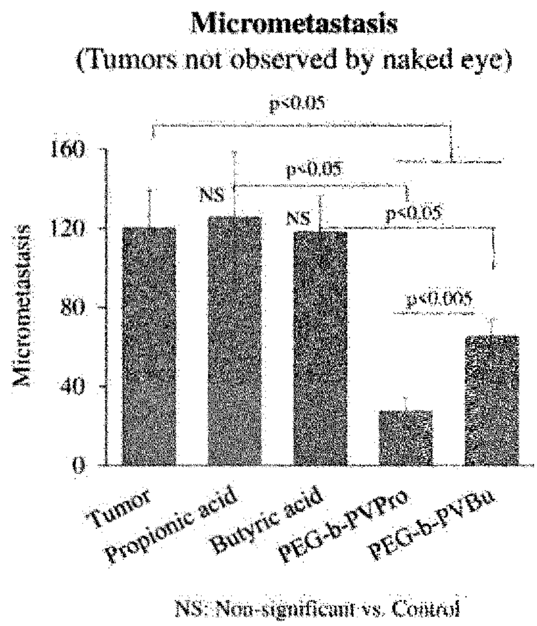
Micrometastasis
(Tumors not observed by naked eye)
p<0.05
p<0.05
p<0.05
p<0.005
NS
NS
160
120
80
40
0
Micrometastasis
Tumor
Propionic acid
Butyric acid
PEG-b-PVPro
PEG-b-PVBu
NS: Non-significant vs. Control

FIG. 11

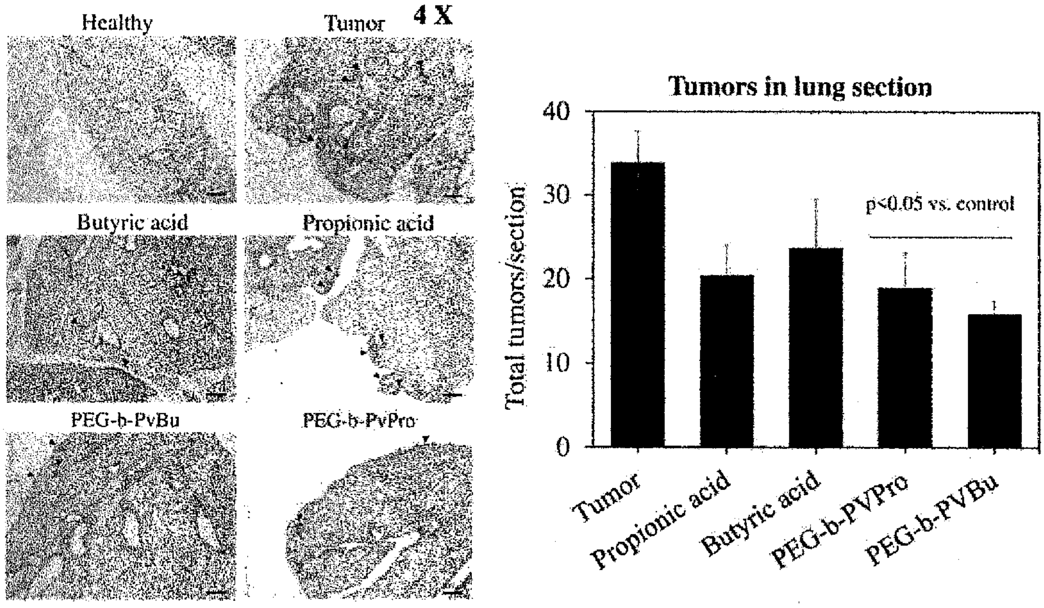
Healthy
Tumor
4 X
Butyric acid
Propionic acid
PEG-b-PvBu
PEG-b-PvPro
Tumors in lung section
Total tumors/section
0
10
20
30
40
p<0.05 vs. control
Tumor
Propionic acid
Butyric acid
PEG-b-PVPro
PEG-b-PVBu

FIG. 12

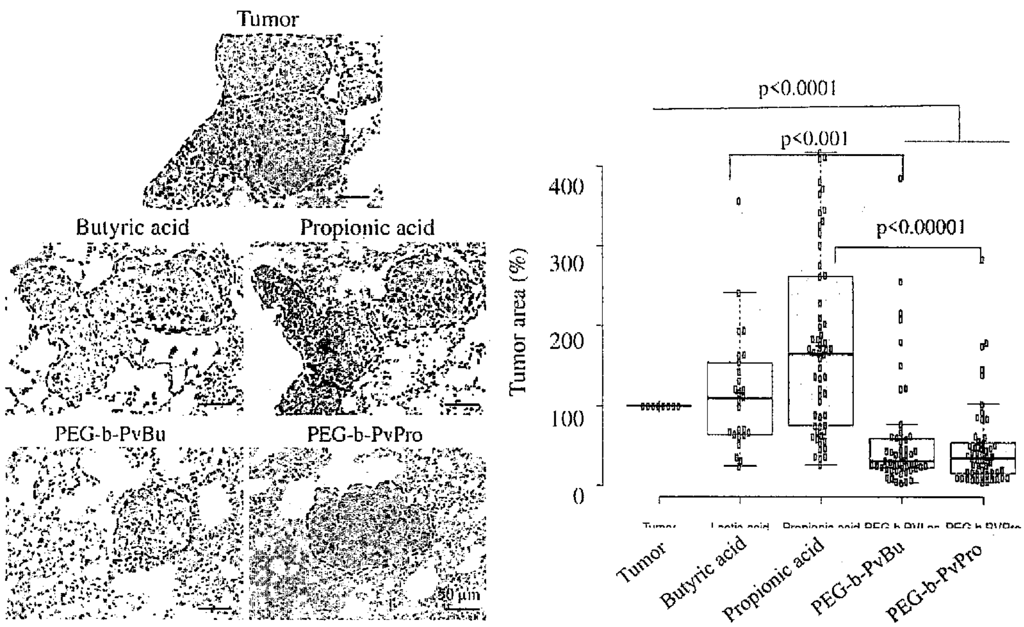

Tumor
Butyric acid
Propionic acid
PEG-b-PvBu
PEG-b-PvPro
50 μm
p<0.0001
p<0.001
p<0.00001
Tumor area (%)
400
300
200
100
0
Tumor
Butyric acid
Propionic acid
PEG-b-PvBu
PEG-b-PvPro


FIG. 13

Sample type: Plasma
Sample volume: 10 uL
Measurement machine: DriChem

No. of samples: 5-7/group

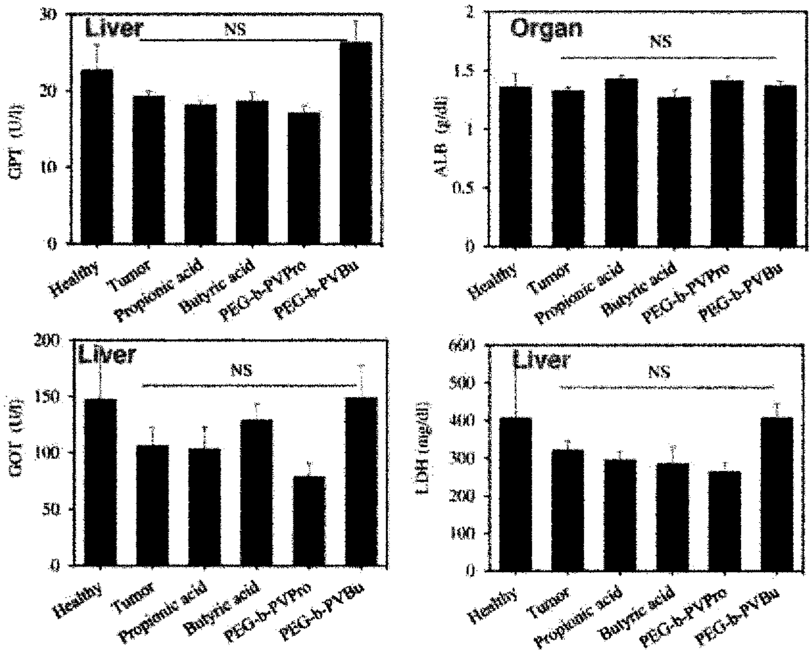


FIG. 14

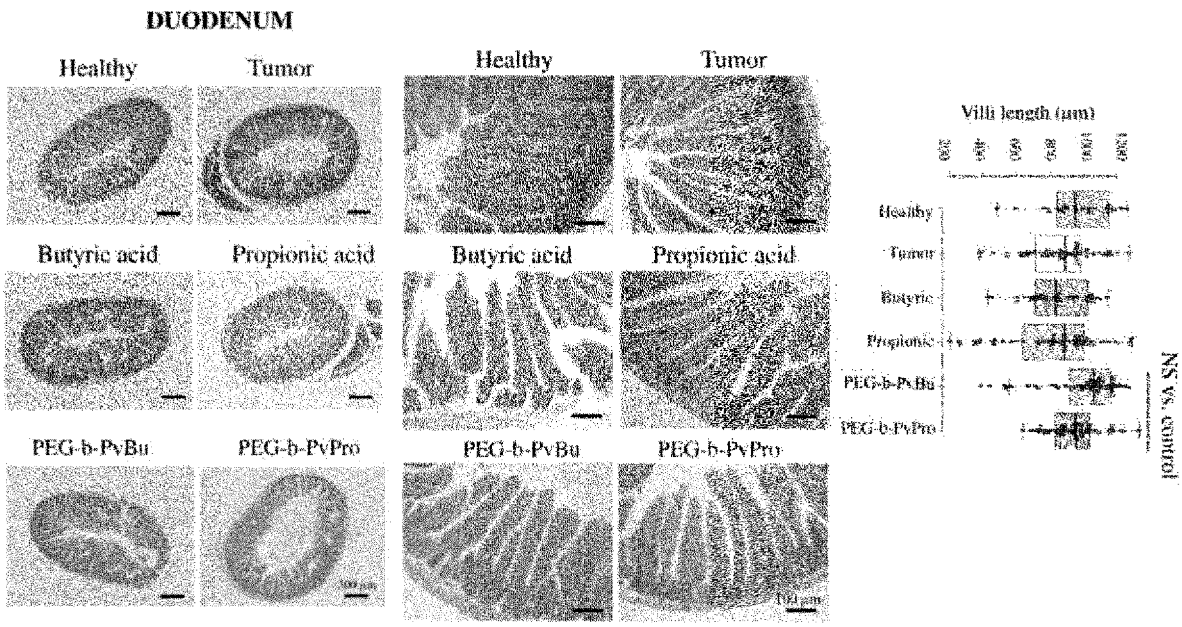
DUODENUM
Healthy
Tumor
Healthy
Tumor
Villi length (μm)
Healthy
Tumor
Butyric
Propionic
PEG-b-PvBu
PEG-b-PvPro
NS vs. control
Butyric acid
Propionic acid
Butyric acid
Propionic acid
PEG-b-PvBu
PEG-b-PvPro
PEG-b-PvBu
PEG-b-PvPro

FIG. 15

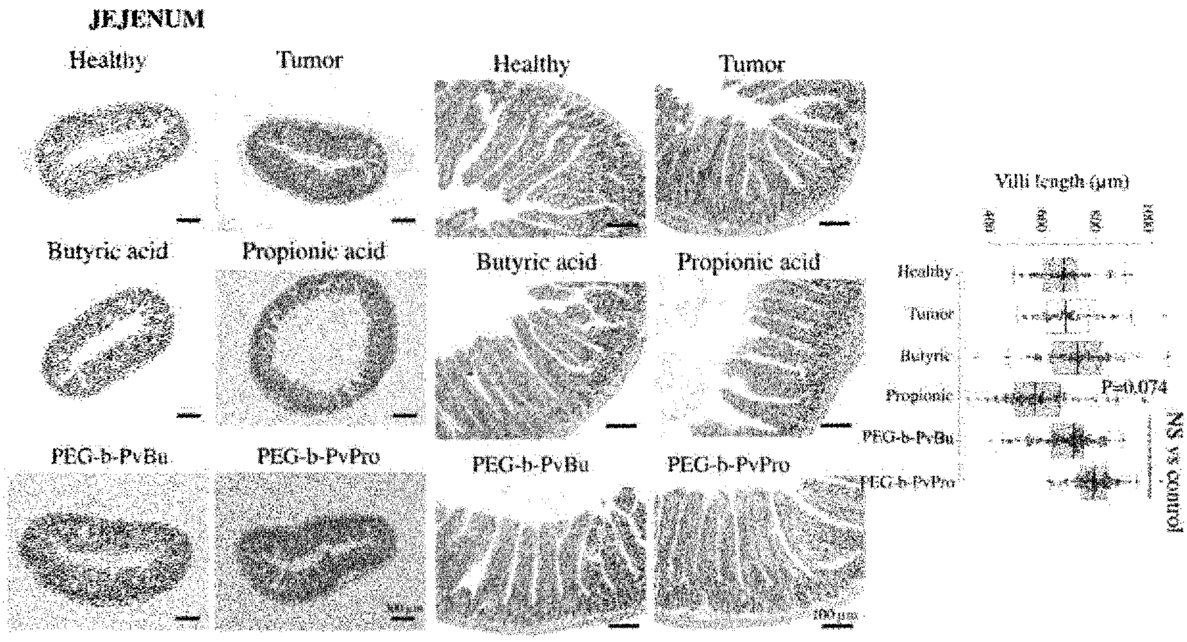
JEJENUM
Healthy
Tumor
Healthy
Tumor
Butyric acid
Propionic acid
Butyric acid
Propionic acid
PEG-b-PvBu
PEG-b-PvPro
PEG-b-PvBu
PEG-b-PvPro
Villi length (μm)
Healthy
Tumor
Butyric
Propionic
PEG-b-PvBu
PEG-b-PvPro
P=0.074
NS vs control

FIG. 16

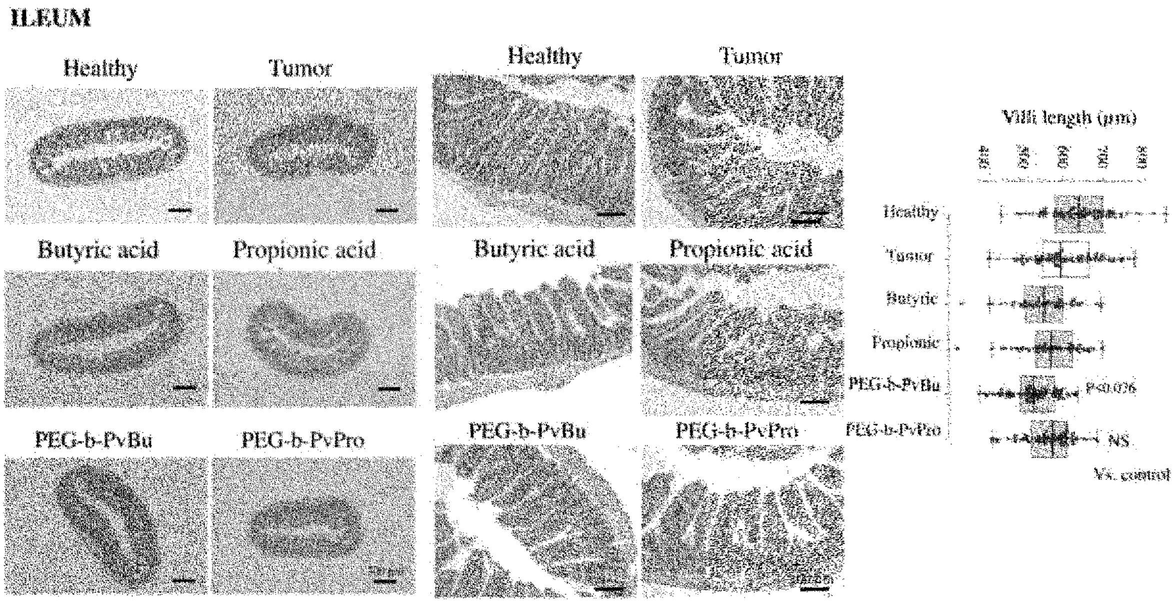
ILEUM
Healthy
Tumor
Butyric acid
Propionic acid
PEG-b-PvBu
PEG-b-PvPro
Healthy
Tumor
Butyric acid
Propionic acid
PEG-b-PvBu
PEG-b-PvPro
Villi length (μm)
Healthy
Tumor
Butyric
Propionic
PEG-b-PvBu
PEG-b-PvPro
P<0.026
NS
Vs. control

FIG. 17

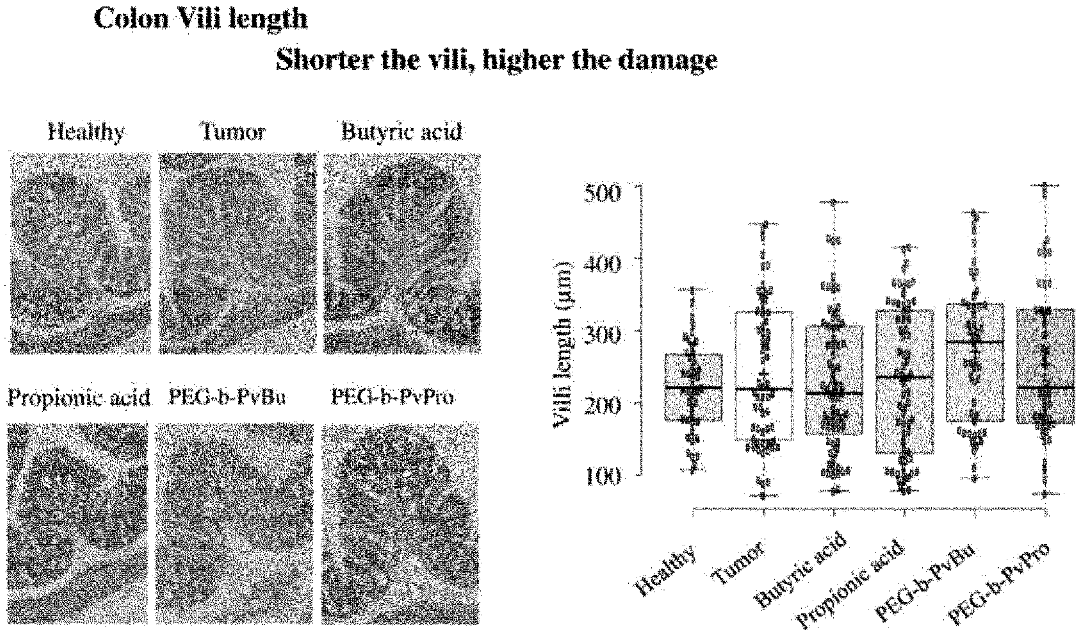
Colon Vili length
Shorter the vili, higher the damage
Healthy
Tumor
Butyric acid
Propionic acid
PEG-b-PvBu
PEG-b-PvPro
Villi length (µm)
500
400
300
200
100
Healthy
Tumor
Butyric acid
Propionic acid
PEG-b-PvBu
PEG-b-PvPro

FIG. 18

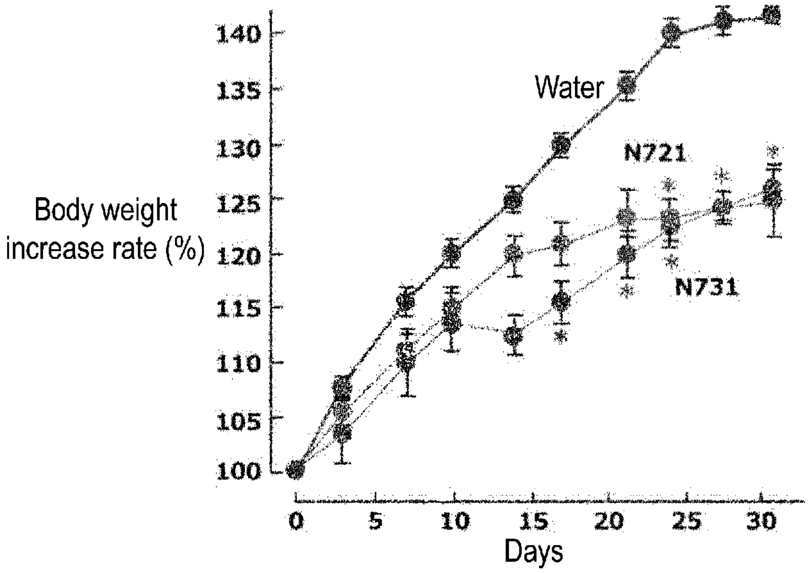
Body weight increase rate (%)
140
135
130
125
120
115
110
105
100
Water
N721
N731
0
5
10
15
20
25
30
Days

FIG. 19

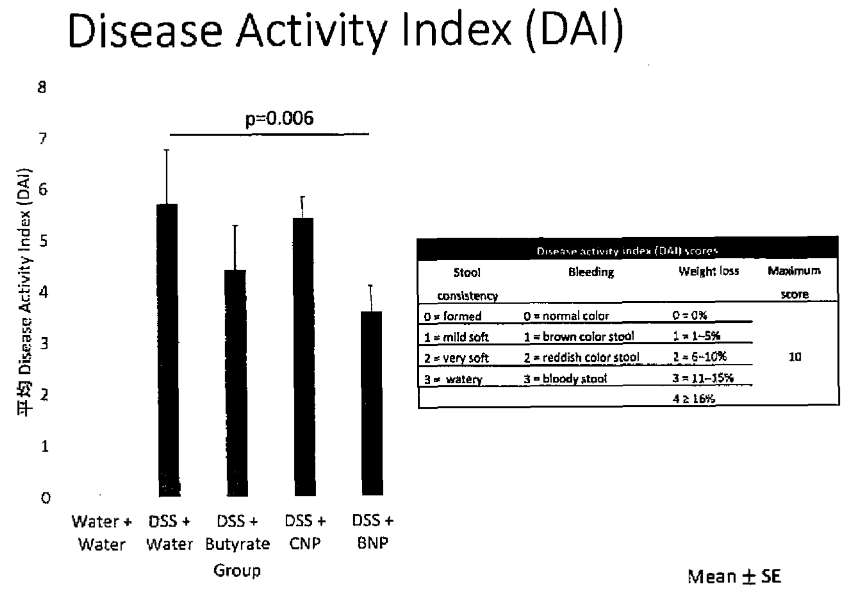


| Disease activity index (DAI) scores | | | |
|---|---|---|---|
| Stool consistency | Bleeding | Weight loss | Maximum score |
| 0 = formed | 0 = normal color | 0 = 0% | 10 |
| 1 = mild soft | 1 = brown color stool | 1 = 1~5% | |
| 2 = very soft | 2 = reddish color stool | 2 = 6~10% | |
| 3 = watery | 3 = bloody stool | 3 = 11~15% | |
| | | 4 ≥ 16% | |

Mean ± SE

FIG. 20

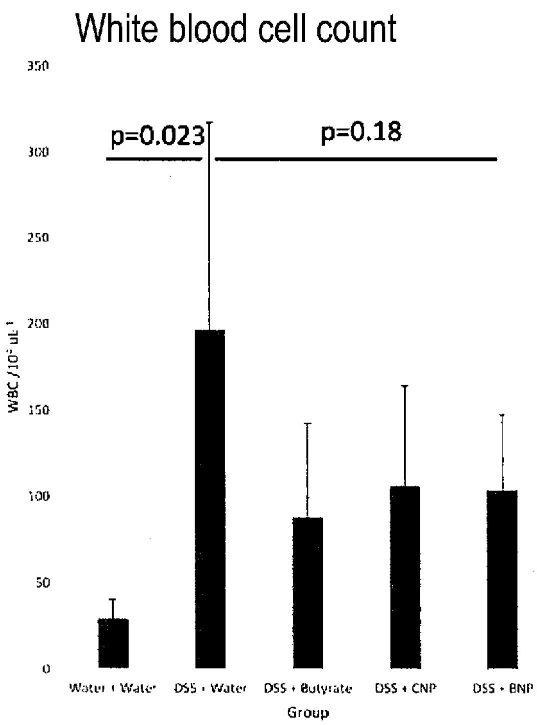


Mean ± SE

FIG. 21

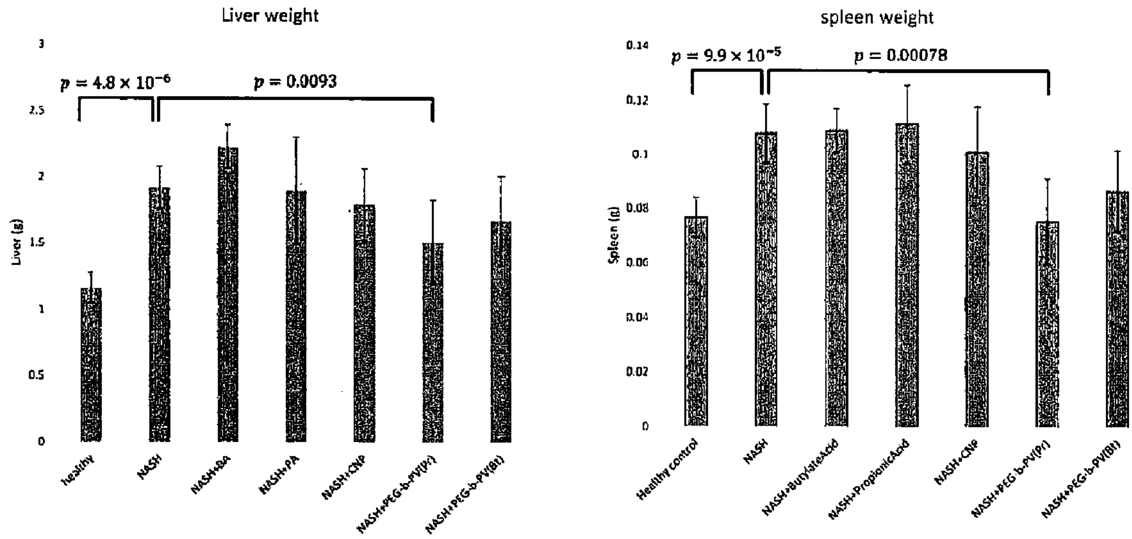
Liver weight
$p = 4.8 \times 10^{-6}$
$p = 0.0093$
Liver (g)
3
2.5
2
1.5
1
0.5
0
healthy
NASH
NASH+BA
NASH+PA
NASH+CNP
NASH+PEG-b-PV(Pr)
NASH+PEG-b-PV(Bt)
spleen weight
$p = 9.9 \times 10^{-5}$
$p = 0.00078$
Spleen (g)
0.14
0.12
0.1
0.08
0.06
0.04
0.02
0
Healthy control
NASH
NASH+ButyrateAcid
NASH+PropionicAcid
NASH+CNP
NASH+PEG-b-PV(Pr)
NASH+PEG-b-PV(Bt)

FIG. 22

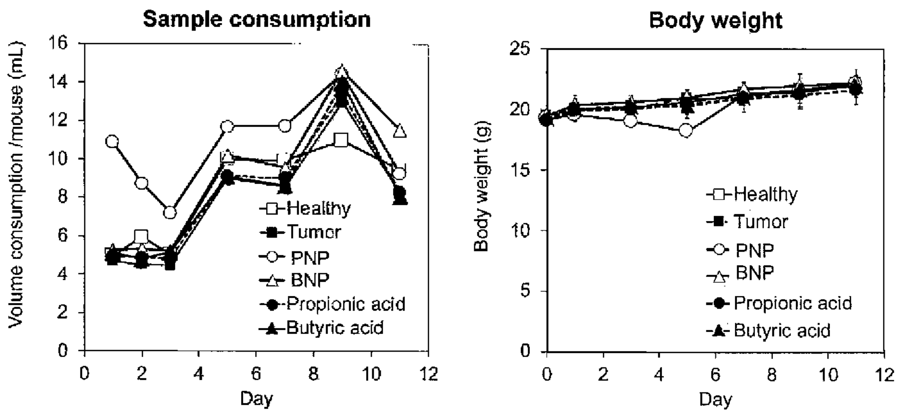
Sample consumption
Volume consumption /mouse (mL)
16
14
12
10
8
6
4
2
0
0 2 4 6 8 10 12
Day
Healthy
Tumor
PNP
BNP
Propionic acid
Butyric acid
Body weight
Body weight (g)
25
20
15
10
5
0
0 2 4 6 8 10 12
Day
Healthy
Tumor
PNP
BNP
Propionic acid
Butyric acid

FIG. 23

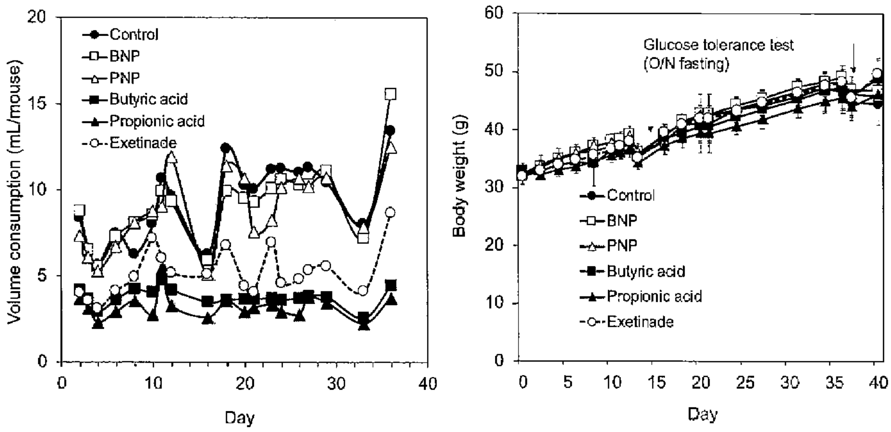
Volume consumption (mL/mouse)
Day
Control
BNP
PNP
Butyric acid
Propionic acid
Exetinade
Body weight (g)
Glucose tolerance test
(O/N fasting)

FIG. 24

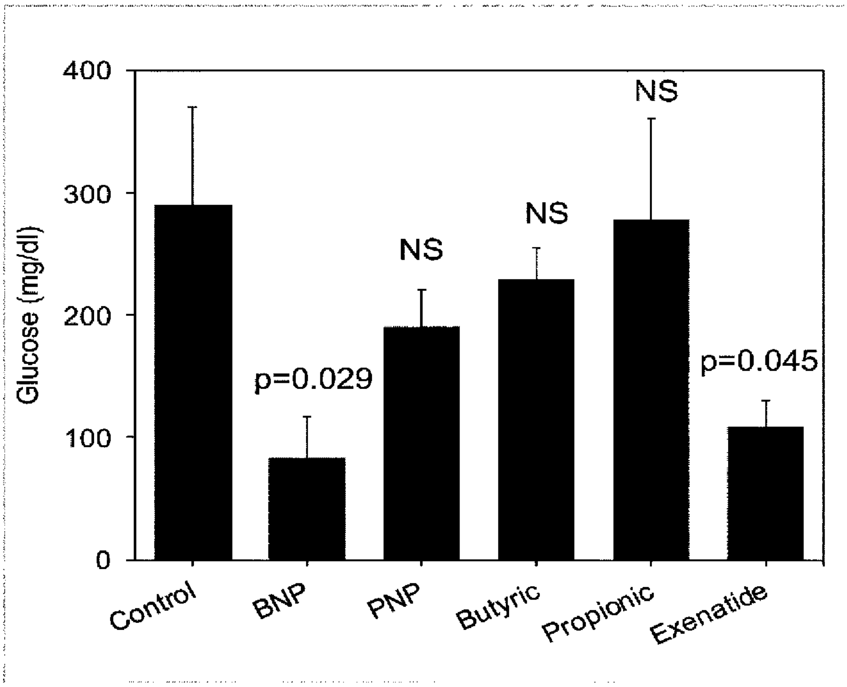
400
300
200
100
0
Glucose (mg/dl)
NS
NS
NS
p=0.029
p=0.045
Control
BNP
PNP
Butyric
Propionic
Exenatide

FIG. 25

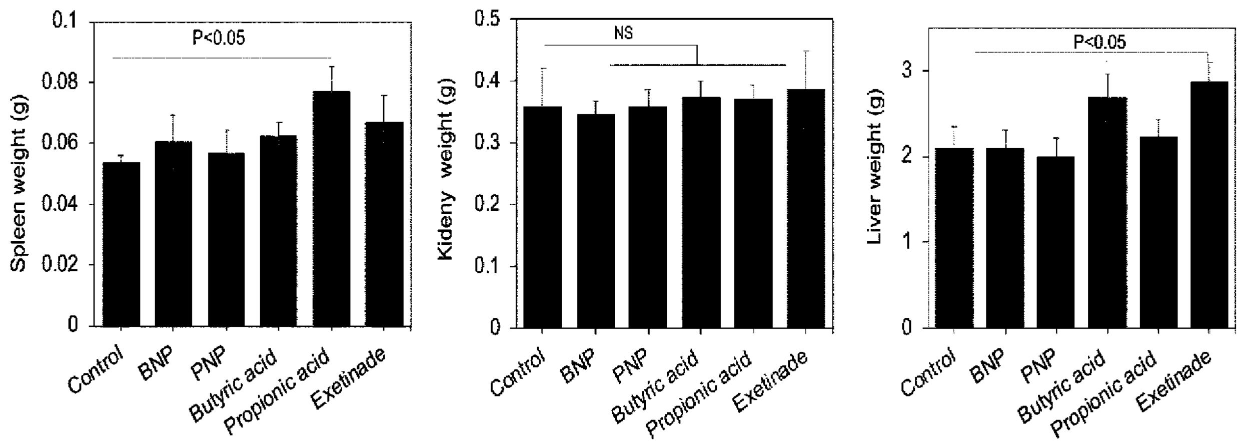
0.1
0.08
0.06
0.04
0.02
0
Spleen weight (g)
P<0.05
Control
BNP
PNP
Butyric acid
Propionic acid
Exetinade
0.5
0.4
0.3
0.2
0.1
0
Kideny weight (g)
NS
Control
BNP
PNP
Butyric acid
Propionic acid
Exetinade
3
2
1
0
Liver weight (g)
P<0.05
Control
BNP
PNP
Butyric acid
Propionic acid
Exetinade

FIG. 26

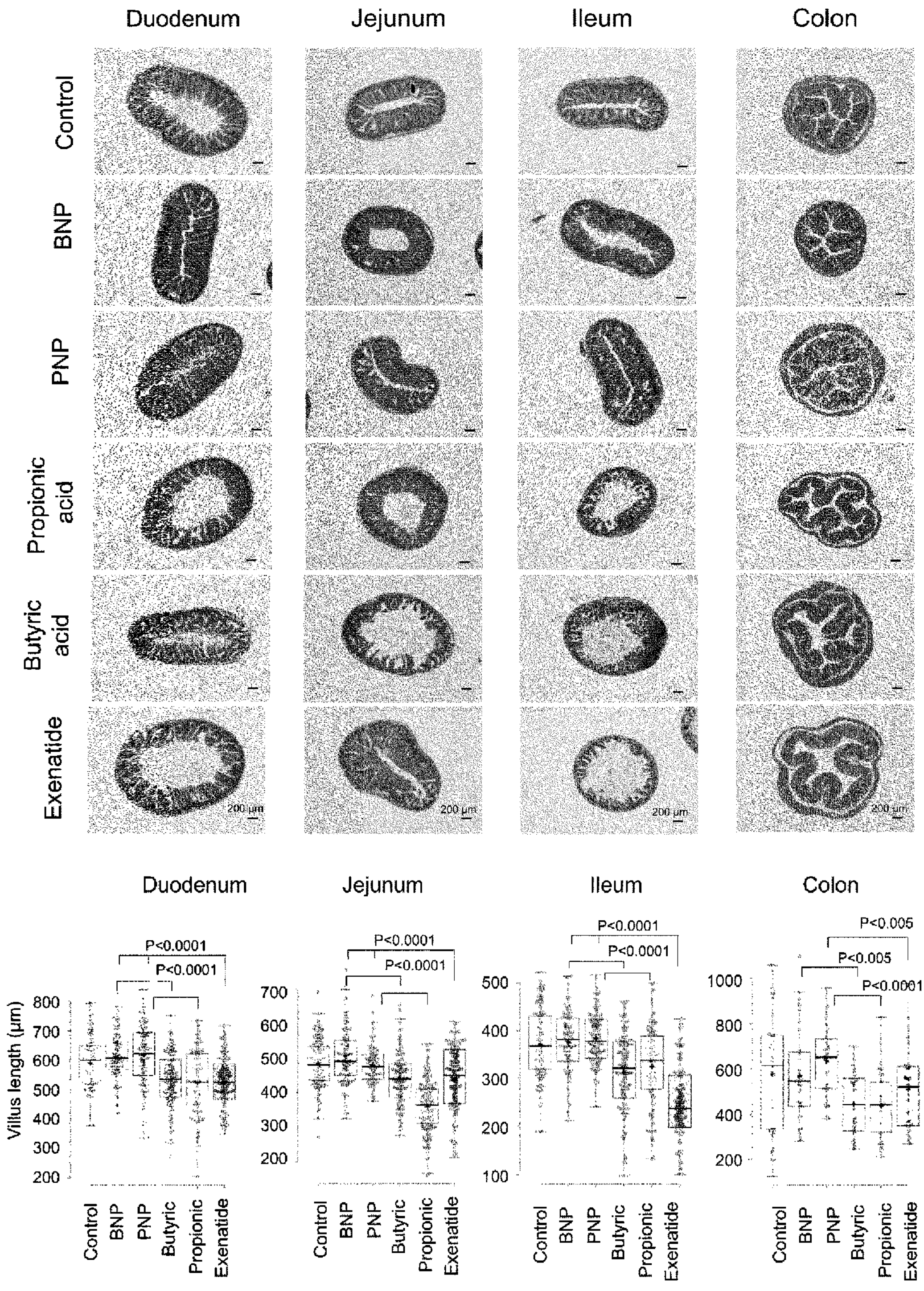
Duodenum
Jejunum
Ileum
Colon
Control
BNP
PNP
Propionic acid
Butyric acid
Exenatide
200 μm
Villus length (μm)
P<0.0001
P<0.005
P<0.0001
Control
BNP
PNP
Butyric
Propionic
Exenatide

FIG. 27

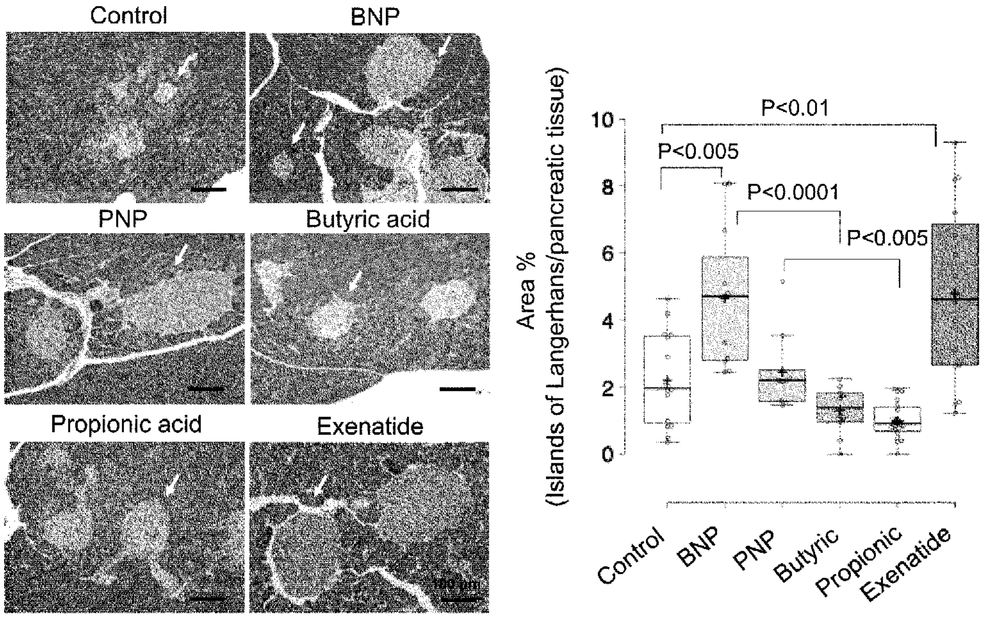


Arrows indicate island of langerhans

FIG. 28

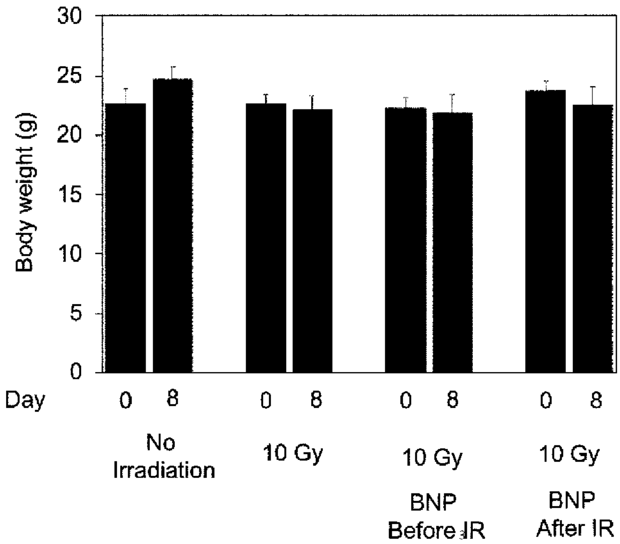
30
25
20
15
10
5
0
Body weight (g)
Day
0 8 0 8 0 8 0 8
No Irradiation
10 Gy
10 Gy
BNP Before IR
10 Gy
BNP After IR

FIG. 29

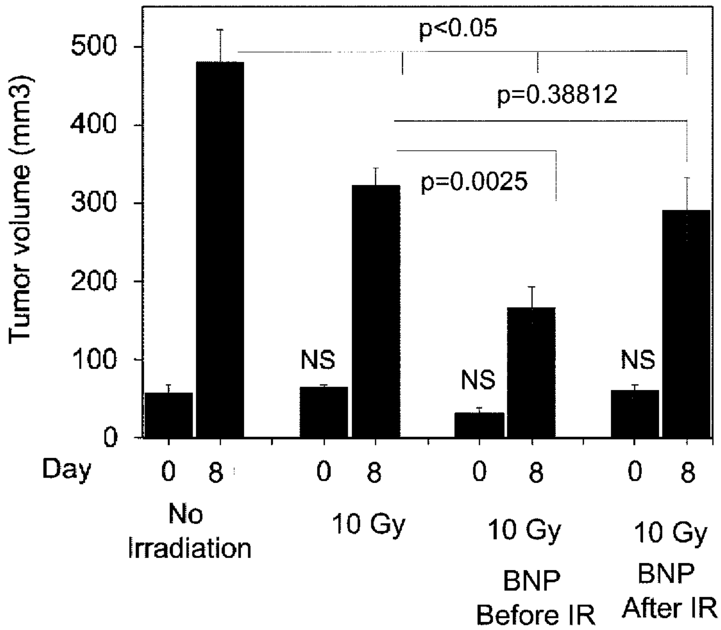


NS: non-significant vs no irradiation at day 0

FIG. 30

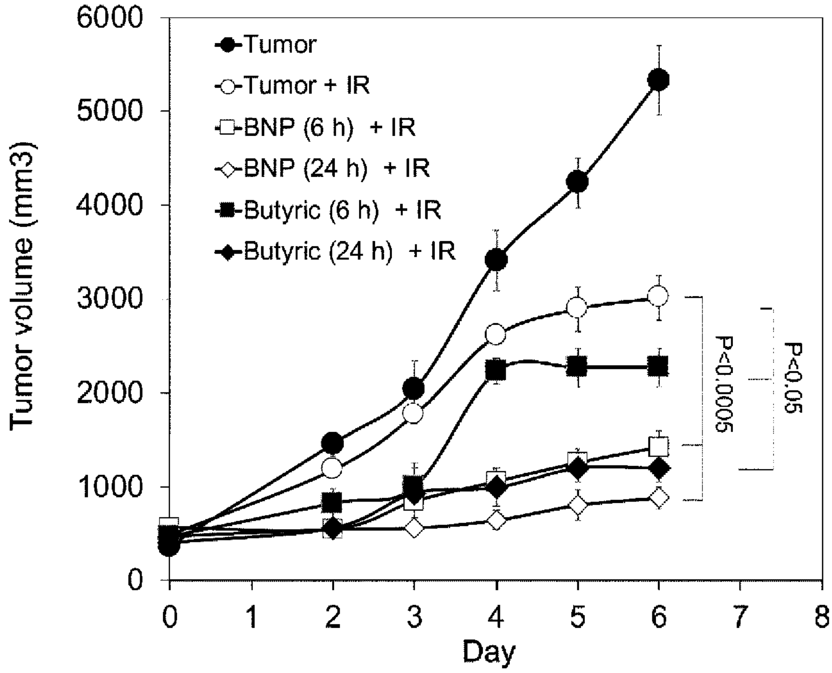
Tumor
Tumor + IR
BNP (6 h) + IR
BNP (24 h) + IR
Butyric (6 h) + IR
Butyric (24 h) + IR
Tumor volume (mm3)
Day
P<0.0005
P<0.05
0
1000
2000
3000
4000
5000
6000
0 1 2 3 4 5 6 7 8

FIG. 31

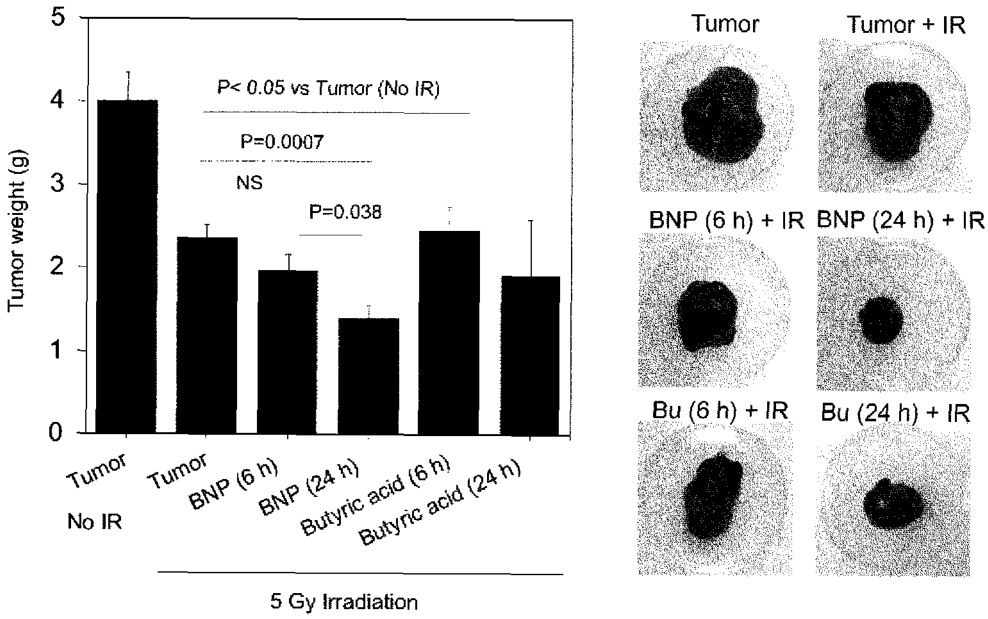

5
4
3
2
1
0
Tumor weight (g)
P< 0.05 vs Tumor (No IR)
P=0.0007
NS
P=0.038
Tumor
No IR
Tumor
BNP (6 h)
BNP (24 h)
Butyric acid (6 h)
Butyric acid (24 h)
5 Gy Irradiation
Tumor
Tumor + IR
BNP (6 h) + IR
BNP (24 h) + IR
Bu (6 h) + IR
Bu (24 h) + IR


FIG. 32

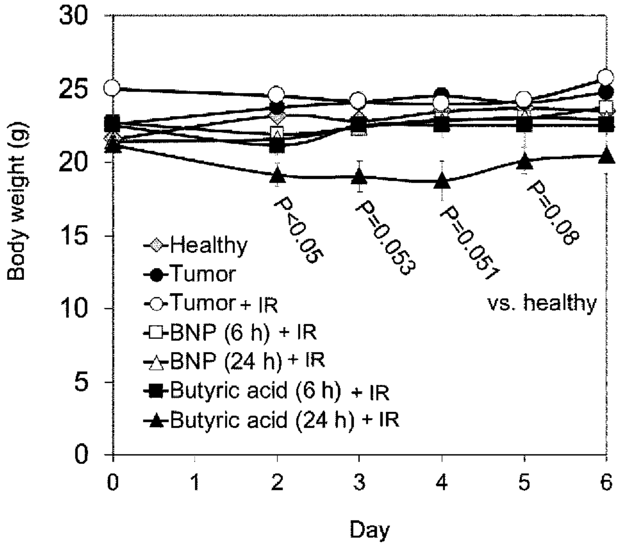
30
25
20
15
10
5
0
Body weight (g)
P<0.05
P=0.053
P=0.051
P=0.08
vs. healthy
Healthy
Tumor
Tumor + IR
BNP (6 h) + IR
BNP (24 h) + IR
Butyric acid (6 h) + IR
Butyric acid (24 h) + IR
0
1
2
3
4
5
6
Day

FIG. 33

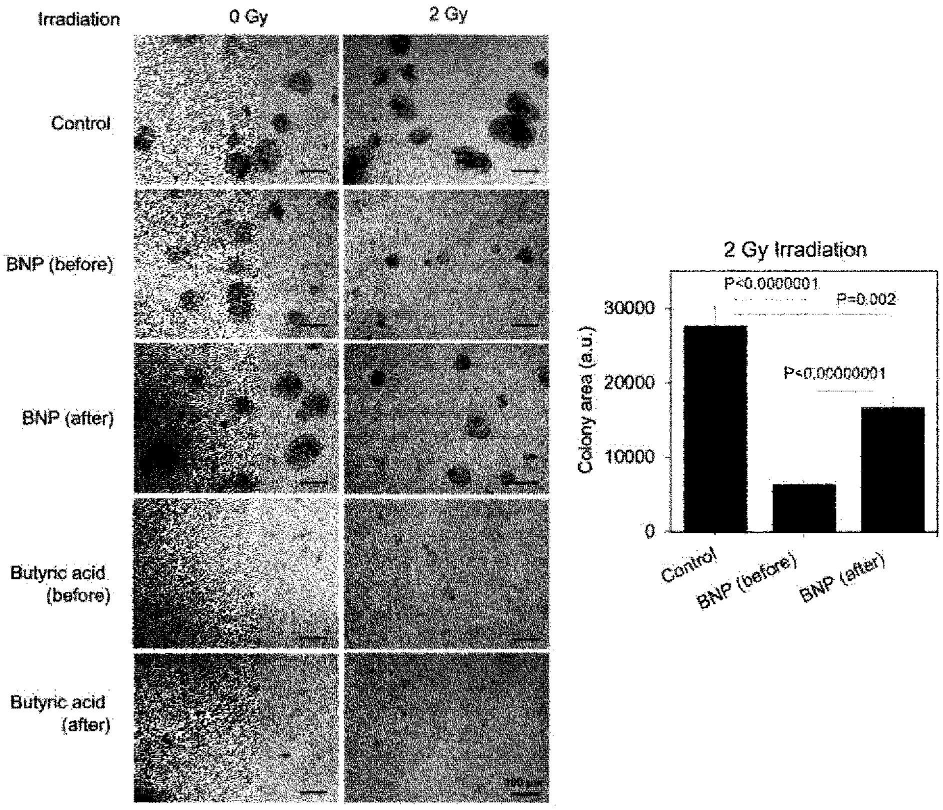
Irradiation
0 Gy
2 Gy
Control
BNP (before)
BNP (after)
Butyric acid (before)
Butyric acid (after)
2 Gy Irradiation
P<0.0000001
P=0.002
P<0.00000001
Colony area (a.u.)
30000
20000
10000
0
Control
BNP (before)
BNP (after)

FIG. 34

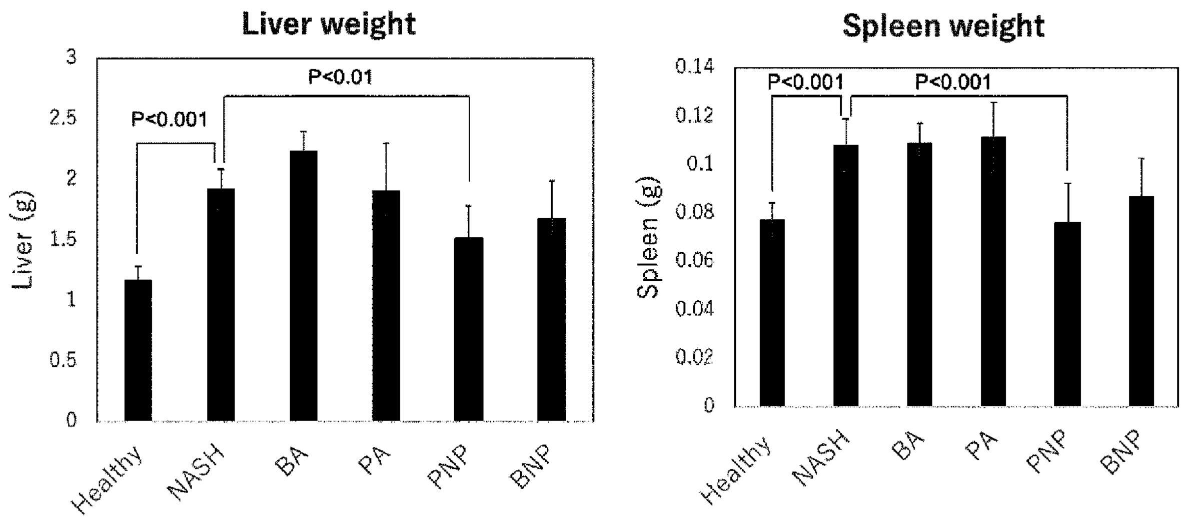
Liver weight
Liver (g)
3
2.5
2
1.5
1
0.5
0
P<0.01
P<0.001
Healthy
NASH
BA
PA
PNP
BNP
Spleen weight
Spleen (g)
0.14
0.12
0.1
0.08
0.06
0.04
0.02
0
P<0.001
P<0.001
Healthy
NASH
BA
PA
PNP
BNP

FIG. 35

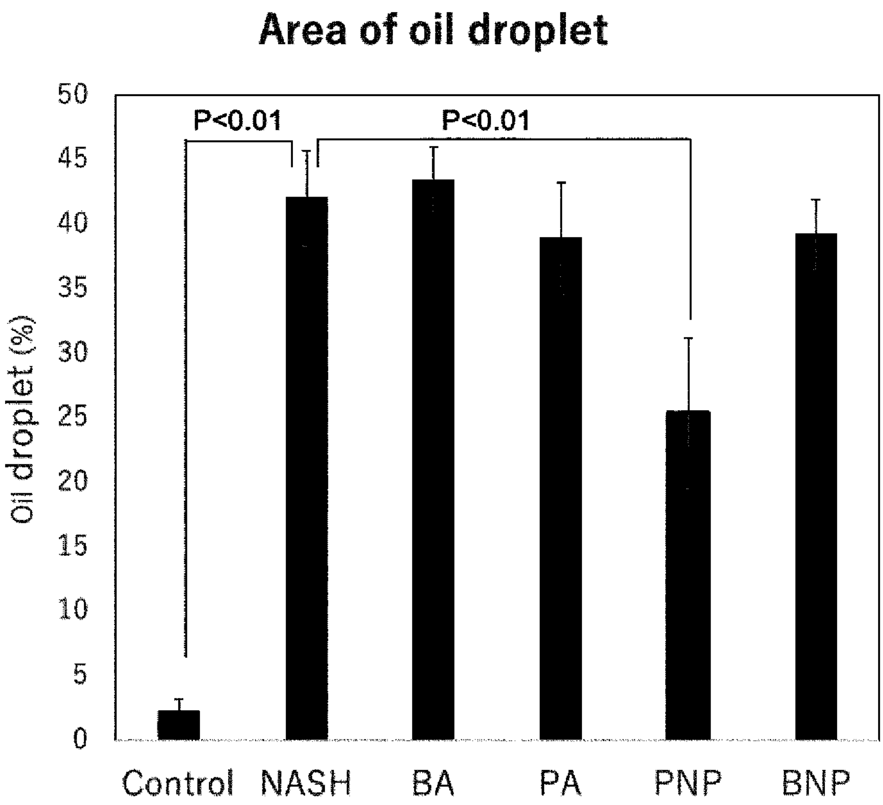
Area of oil droplet
P<0.01
P<0.01
50
45
40
35
30
25
20
15
10
5
0
Oil droplet (%)
Control
NASH
BA
PA
PNP
BNP

FIG. 36

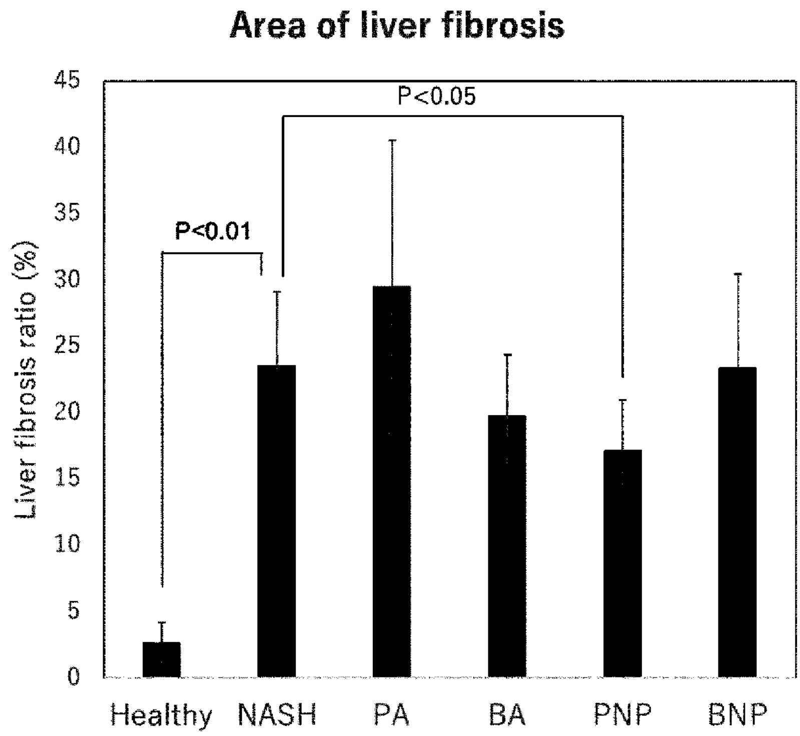
Area of liver fibrosis
45
40
35
30
25
20
15
10
5
0
Liver fibrosis ratio (%)
P<0.05
P<0.01
Healthy
NASH
PA
BA
PNP
BNP

FIG. 37

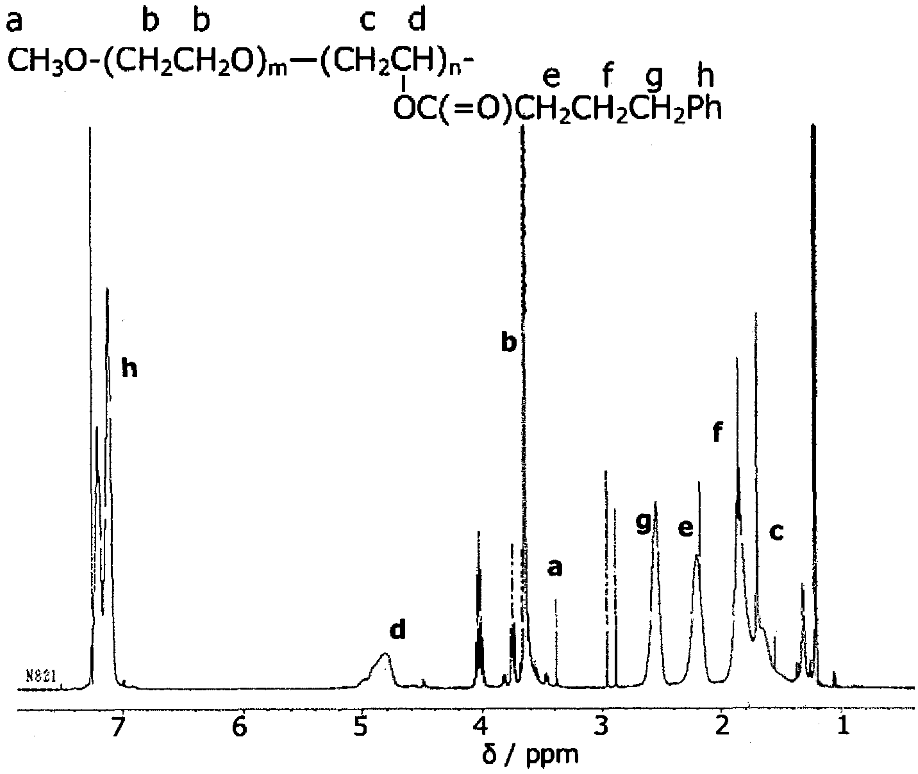
a b b c d
$CH_3O-(CH_2CH_2O)_m-(CH_2CH)_n-$
e f g h
$OC(=O)CH_2CH_2CH_2Ph$
h
b
a
d
g
e
f
c
N821
7
6
5
4
3
2
1
δ / ppm

FIG. 38

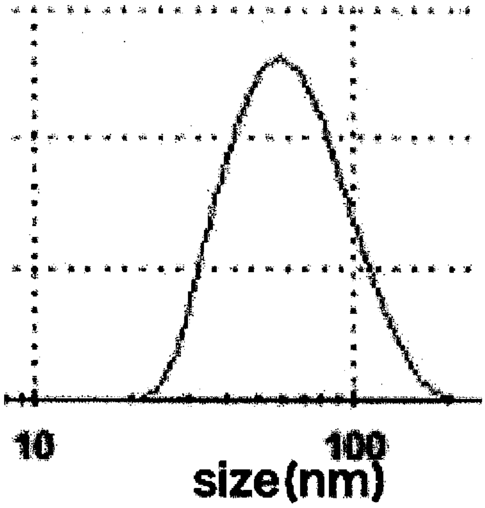


FIG. 39

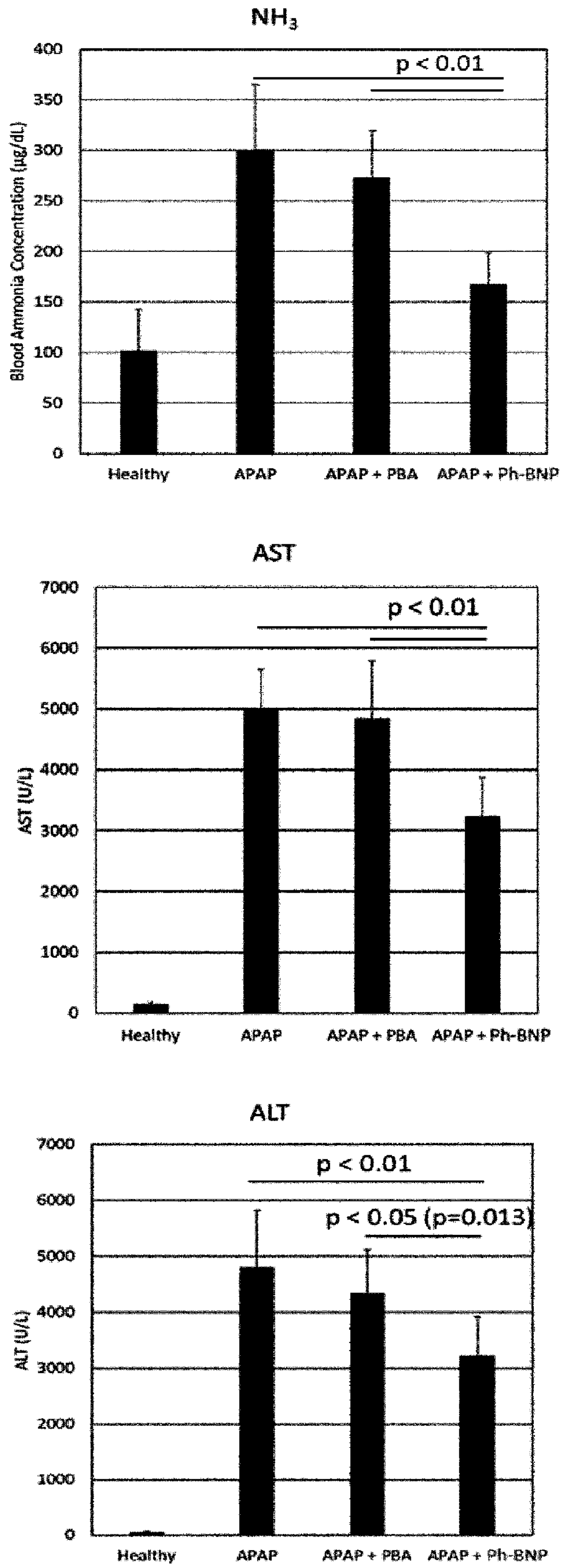

NH3
400
350
300
250
200
150
100
50
0
Blood Ammonia Concentration (μg/dL)
p < 0.01
Healthy
APAP
APAP + PBA
APAP + Ph-BNP
AST
7000
6000
5000
4000
3000
2000
1000
0
AST (U/L)
p < 0.01
Healthy
APAP
APAP + PBA
APAP + Ph-BNP
ALT
7000
6000
5000
4000
3000
2000
1000
0
ALT (U/L)
p < 0.01
p < 0.05 (p=0.013)
Healthy
APAP
APAP + PBA
APAP + Ph-BNP


FIG. 40

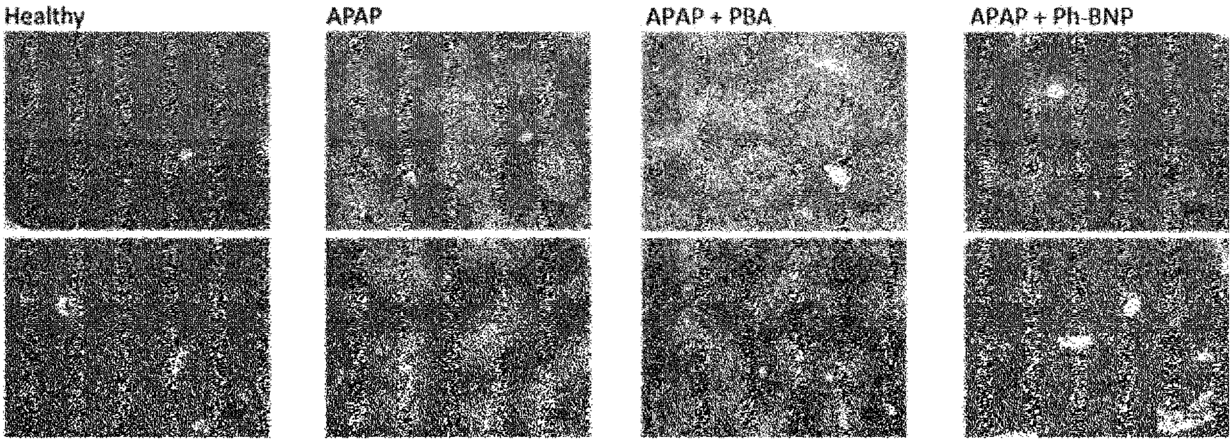


FIG. 41

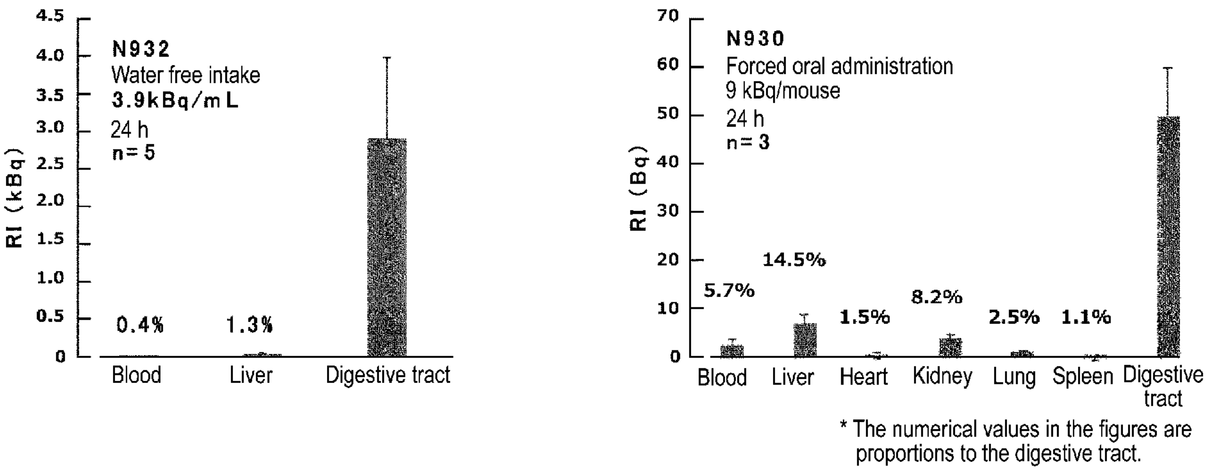
4.5
4.0
3.5
3.0
2.5
2.0
1.5
1.0
0.5
0
RI (kBq)
N932
Water free intake
3.9kBq/mL
24 h
n= 5
0.4%
1.3%
Blood
Liver
Digestive tract
70
60
50
40
30
20
10
0
RI (Bq)
N930
Forced oral administration
9 kBq/mouse
24 h
n= 3
5.7%
14.5%
1.5%
8.2%
2.5%
1.1%
Blood
Liver
Heart
Kidney
Lung
Spleen
Digestive tract
* The numerical values in the figures are proportions to the digestive tract.

FIG. 42

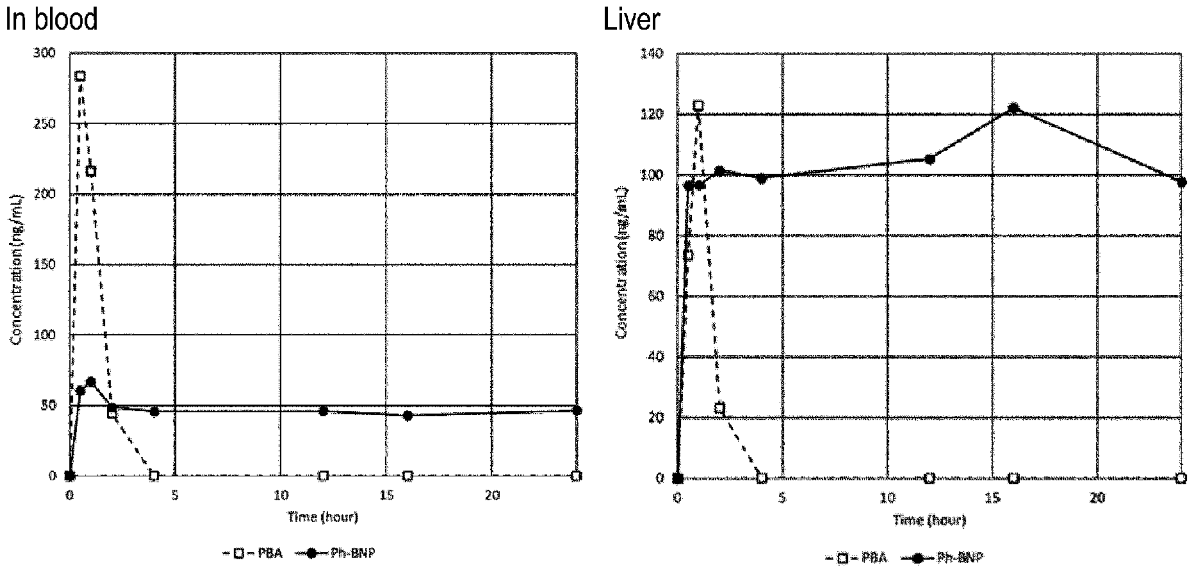
In blood
300
250
200
150
100
50
0
Concentration (ng/mL)
0
5
10
15
20
Time (hour)
PBA
Ph-BNP
Liver
140
120
100
80
60
40
20
0
Concentration (ng/mL)
0
5
10
15
20
Time (hour)
PBA
Ph-BNP

FIG. 43

**INTERNATIONAL SEARCH REPORT**

International application No. PCT/JP2020/029239

A. CLASSIFICATION OF SUBJECT MATTER

K61P 1/04(2006.01)i; A61P 1/16(2006.01)i; A61P 3/04(2006.01)i; A61P 35/00(2006.01)i; C08F 8/14(2006.01)i; C08F 290/06(2006.01)i; C08C 65/334(2006.01)i; C08F 293/00(2006.01)i; A61K 47/60(2017.01)i; A61K 31/19(2006.01)i

FI: C08F293/00; C08C65/334; A61K31/19; A61P1/04; A61P3/04; A61P1/16; A61P35/00; C08F290/06; C08F8/14; A61K47/60

According to International Patent Classification (IPC) or to both national classification and IPC

B. FIELDS SEARCHED

Minimum documentation searched (classification system followed by classification symbols)

A61P1/04; A61P1/16; A61P3/04; A61P35/00; C08FS/14; C08F290/06; C08G65/334; C08F293/00; A61K47/60; A61K31/19

Documentation searched other than minimum documentation to the extent that such documents are included in the fields searched

A61P1/04; A61P1/16; A61P3/04; A61P35/00; C08FS/14; C08F290/06; C08G65/334; C08F293/00; A61K47/60; A61K31/19

Electronic data base consulted during the international search (name of data base and, where practicable, search terms used)

CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 6-16752 A (HUELS AKTIENGESELLSCHAFT) 25.01.1994 (1994-01-25) claims, paragraphs [0029]-[0052] | 1-4,7<br>5,6,8-10 |
| X<br>A | JP 6-16747 A (KURARAY CO., LTD.) 25.01.1994 (1994-01-25) claims, paragraphs [0012], [0016]-[0027], [0048]-[0068] | 1-4,7<br>5,6,8-10 |
| X<br>A | JP 2007-185494 A (CORDIS CORPORATION) 26.07.2007 (2007-07-26) claims | 1,2,4,7<br>3,5,6,8-10 |
| A | WO 2018/135592 A1 (UNIVERSITY OF TSUKUBA) 26.07.2018 (2018-07-26) | 1-10 |
| A | JP 2019-1786 A (UNIVERSITY OF TSUKUBA) 10.01.2019 (2019-01-10) | 1-10 |

☒ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 02 October 2020 (02.10.2020) | 20 October 2020 (20.10.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/029239

C (Continuation). DOCUMENTS CONSIDERED TO BE RELEVANT

| Category* | Citation of document, with indication, where appropriate, of the relevant passages | Relevant to claim No. |
|---|---|---|
| P,A | WO 2019/208017 A1 (UNIVERSITY OF TSUKUBA, ASAHIKAWA MEDICAL UNIVERSITY) 31.10.2019 (2019-10-31) | 1-10 |

Form PCT/ISA/210 (continuation of second sheet) (January 2015)

**INTERNATIONAL SEARCH REPORT**
Information on patent family members

International application No.
PCT/JP2020/029239

| Patent Documents referred in the Report | Publication Date | Patent Family | Publication Date |
|---|---|---|---|
| JP 6-16752 A | 25 Jan. 1994 | US 5385971 A<br>claims, column 5, line 7 to column 8, line 24<br>US 5516836 A<br>EP 565825 A1<br>DE 4212768 A1<br>FI 931635 A | |
| JP 6-16747 A | 25 Jan. 1994 | (Family: none) | |
| JP 2007-185494 A | 26 Jul. 2007 | US 2007/0122443 A1<br>claims<br>EP 1790702 A1<br>CA 2569162 A<br>CA 2569162 A1 | |
| WO 2018/135592 A1 | 26 Jul. 2018 | US 2019/0345293 A1<br>EP 3572443 A1 | |
| JP 2019-1786 A | 10 Jan. 2019 | (Family: none) | |
| WO 2019/208617 A1 | 31 Oct. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009133647 A
- WO 2016052463 A


### Non-patent literature cited in the description


- **TAKASHI SAKATA ; HIROHUMI ICHIKAWA.** Physiological Actions of Short-Chain Fatty Acids. *Journal of Japan Oil Chemists' Society,* 1997, vol. 46, 1205-1212